(19) Europäisches Patentamt / European Patent Office / Office européen des brevets

(11) **EP 3 720 876 B1**

(12) **EUROPEAN PATENT SPECIFICATION**

(45) Date of publication and mention
of the grant of the patent:
**05.02.2025 Bulletin 2025/06**

(21) Application number: **18826506.0**

(22) Date of filing: **04.12.2018**

(51) International Patent Classification (IPC):
**C07K 16/00** *(2006.01)*     **C07K 14/00** *(2006.01)*
**C12P 21/02** *(2006.01)*     **G01N 33/68** *(2006.01)*

(52) Cooperative Patent Classification (CPC):
**C07K 16/00; C07K 14/00; C12P 21/02;**
**C40B 30/04;** C07K 2317/622; C07K 2317/92;
C07K 2317/94

(86) International application number:
**PCT/EP2018/083508**

(87) International publication number:
**WO 2019/110596 (13.06.2019 Gazette 2019/24)**

(54) **METHOD FOR SCREENING THE ACTIVITY OF POLYPEPTIDES PRODUCED BY CELL-FREE IN VITRO TRANSLATION**

VERFAHREN ZUM AKTIVITÄTSCREENING VON POLYPEPTIDEN, DIE DURCH ZELLFREIE IN VITRO-TRANSLATION HERGESTELLT WURDEN

PROCÉDÉ DE CRIBLAGE DE L'ACTIVITÉ DE POLYPEPTIDES PRODUIT PAR TRANSLATION IN VITRO SANS CELLULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.12.2017 GB 201720369**

(43) Date of publication of application:
**14.10.2020 Bulletin 2020/42**

(73) Proprietor: **ProImmune Limited**
**Oxford, Oxfordshire OX4 4GA (GB)**

(72) Inventors:
• **SCHWABE, Nikolai**
**Oxford**
**Oxfordshire OX4 4GA (GB)**
• **WILKINS, Sarah**
**Oxford**
**Oxfordshire OX4 4GA (GB)**

(74) Representative: **Vossius & Partner**
**Patentanwälte Rechtsanwälte mbB**
**Siebertstrasse 3**
**81675 München (DE)**

(56) References cited:
**WO-A1-2017/059095     WO-A1-2019/023504**

• **CAPECE MARK C. ET AL: "A simple real-time assay for in vitro translation", RNA, vol. 21, no. 2, 1 February 2015 (2015-02-01), US, pages 296 - 305, XP055828824, ISSN: 1355-8382, Retrieved from the Internet <URL:https://www.ncbi.nlm. nih.gov/pmc/articles/PMC4338355/pdf/296.pdf> DOI: 10.1261/rna.047159.114**
• **STECH M. ET AL: "Cell-free synthesis of functional antibodies using a coupled in vitro transcription-translation system based on CHO cell lysates", SCIENTIFIC REPORTS, vol. 7, no. 1, 1 December 2017 (2017-12-01), XP055828830, Retrieved from the Internet <URL:https://www. nature.com/articles/s41598-017-12364-w.pdf> DOI: 10.1038/s41598-017-12364-w**
• **MATSUNAGA SATOKO ET AL: "A cell-free enzymatic activity assay for the evaluation of HIV-1 drug resistance to protease inhibitors", FRONTIERS IN MICROBIOLOGY, vol. 6, 1220, 31 October 2015 (2015-10-31), Lausanne, XP055828839, ISSN: 1664-302X, DOI: 10.3389/ fmicb.2015.01220**

- SPARKS SUSAN E. ET AL: "Use of a cell-free system to determine UDP-N-acetylglucosamine 2-epimerase and N-acetylmannosamine kinase activities in human hereditary inclusion body myopathy", GLYCOBIOLOGY, vol. 15, no. 11, 1 November 2005 (2005-11-01), US, pages 1102 - 1110, XP055828855, ISSN: 0959-6658, DOI: 10.1093/glycob/cwi100
- ZAHND C ET AL: "Ribosome display: selecting and evolving proteins in vitro that specifically bind to a target", NATURE METHODS, NATURE PUBLISHING GROUP US, NEW YORK, vol. 4, no. 3, 1 March 2007 (2007-03-01), pages 269 - 279, XP002639209, ISSN: 1548-7091, [retrieved on 20070227], DOI: 10.1038/NMETH1003
- ERIK D. CARLSON ET AL: "Cell-free protein synthesis: Applications come of age", BIOTECHNOLOGY ADVANCES, vol. 30, no. 5, 1 September 2012 (2012-09-01), pages 1185 - 1194, XP055072004, ISSN: 0734-9750, DOI: 10.1016/ j.biotechadv.2011.09.016
- YIN GANG ET AL: "Aglycosylated antibodies and antibody fragments produced in a scalable in vitro transcription-translation system", vol. 4, no. 2, 1 March 2012 (2012-03-01), pages 217 - 225, XP002711741, Retrieved from the Internet <URL:https://www.landesbioscience. com/journals/mabs/article/19202/> DOI: 10.4161/ MABS.4.2.19202
- DELLA DUCATA D. ET AL.: "Solution Equilibrium Titration for High-Throughput Affinity Estimation of Unpurified Antibodies and Antibody Fragments.", J. BIOMOL. SCREEN., vol. 20, no. 10, 15 July 2015 (2015-07-15), pages 1256 - 1267, XP002789360
- OJIMA-KATO T., NAGAI S. & NAKANO H.: "Ecobody technology: rapid monoclonal antibody screening method from single B cells using cell-free protein synthesis for antigen-binding fragment formation.", SCIENTIFIC REPORTS, vol. 7, no. 1, 13979, 25 October 2017 (2017-10-25), XP002789361
- HUBER T. ET AL.: "In vitro selection and characterization of DARPins and Fab fragments for the co-crystallization of membrane proteins: The Na+-citrate symporter CitS as an example.", J. STRUCT. BIOL., vol. 159, 2007, pages 206 - 221, XP002789362
- TSAI Y., JIN Z. AND JOHNSON K.A.: "Site-specific labelng of T7 DNA polymerase with a conformationally sensitive fluorophore and its use in detecting single-nucleotirde polymorphism", ANALYTICAL BIOCHEM., vol. 384, 2008, pages 136 - 144, XP002789363
- HOULIHAN G.: "An experimental framework for improved selection of bindingproteins using SNAP display", J. IMMUNOL. METHD., vol. 405, 2014, pages 47 - 56, XP002789364

**Description**

**Field of the invention**

The present invention is in the field of identifying proteins for commercial applications, more in particular in the field of screening of polypeptides from a mutant library, and, equally for producing a polypeptide with a desired activity by said screening for identifying therapeutically relevant polypeptides with improved characteristics.

**Background Art**

Recognition and binding of ligands regulate almost all biological processes, such as immune recognition, cell signaling and communication, transcription and translation, intracellular signalling, and catalysis, i.e., enzyme reactions. Understanding, which peptides in a protein sequence can bind to a given interaction member is of considerable interest for engineering and developing new generations of therapeutic proteins and peptides and in order to design and monitor the effectiveness of therapeutic products. The engineering and development of new generation proteins for commercial applications, including without limitation therapeutic, diagnostic, cosmetic, crop science, consumer product, scientific and other industrial applications comprising for example antibodies, enzymes, hormones, growth factors, neuroactive molecules, and immune epitopes, has been a longstanding interest in the art (Patents US510240A; WO201497113 A2; Tobin et al., 2014, Curr. Drug Metab. 15(7): 73-756; Popplewell, 2015, ADME and Translational Pharmacokinetics/Pharmacodynamics of Therapeutic Proteins). A wide range of screening techniques based on a procedure to express a polypeptide and to detect the interaction between a polypeptide are know in the art (Nieri et al, Current Clinical Medicine, 16: 753-779 (2009); Rajpal et al, Proc. Natl. Acad. Sci., 102: 8466-8471 (2005); Dubel et al, Trends Biotechnology, 28: 333-339 (2010); Igawa et al, mAbs, 3(3): 243-252 (2011); Bostrom et al, Science, 323: 1610-1614 (2009); (Mason et al., Trends Mol. Med. 8: 324-329 (2002); Wurm an Bernard, Curr. Opin.Biotechnol. 10: 156-159 (1999); Zoller, Cur. Opin. Biotechnol. 2:526-531); Brannigan and Wilkinson, Nature Rev. Mol. CEllBiol. 3: 964-970 (2002); Watson et al., Recombinant DNA, Scientific American Books, New York, 453-470 (1992)).

A routinely used approach for generating protein candidates for commercial applications encompasses the combination of (a) constructing a mutant library from an existing protein and (b) screening library members by means of a range of different affinity strategies until better performing polypeptides, including modified affinities, increased stability, reduced immunogenicity, and the like, are found (Igawa et al, mAbs, 3(3): 243-252 (2011); Bostrom et al, Science, 323: 1610-1614 (2009)). In addition to affinity assays, enzyme assays may be of particular interest for engineering and development of new generation enzymes, or substrates or co-factors thereof with increased activity for commercial applications. However, for example, enzymes isolated from different species, e.g. from bacteria, are expected to have a high immunogenicity when applied to a human subject. Procedures for enzyme assays are documented or cited in various standard books (Bergmeyer, Verlag Chemie, Grundlagen der enzymatischen Analyse (1977); Bergmeyer, Verlag Chemie, Methods of Enzymatic Analysis, 3rd ed. (1983); Bisswanger, Wiley-VCH, Enzyme Kinetics, Principles and Methods, 2nd ed. (2008); Bisswanger, Wiley-Blackwell, Practical Enzymology, 2nd ed. (2011); Schomburg, Springer, Springer Handbook of Enzymes, 2nd ed. (2009); Brenda database (www.brenda-enzymes.org); ExPASy database (www.wxpasy.org/enzymes)). For evaluating the activity of an enzyme, or the activity of an enzyme by its interaction with variants of a co-factor thereof, or of a regulatory protein thereof, or of a substrate thereof, the enzyme initial velocity may be determined. Thereby, for example, a fixed concentration of enzyme is incubated with varying concentrations of substrate (and co-factors or regulatory proteins of the enzyme reaction, where applicable) whereas the product formed at different substrate concentrations is monitored and plotted as a progress curve over time. As the catalyzed reaction initially follows a linear relationship, the velocity ($V_0$) may be determined from the slope of the linear part of the progress curve for each substrate concentration and is referred to as the number of moles of product formed within a distinct time unit (1 min or 1 s). Then, the reaction velocity may be plotted as a function of the concentration of substrate, or regulatory protein, or cofactor of the enzyme under study. Thereby, the relationship between the reaction velocity and the substrate concentrations follows a Michaelis-Menten kinetic, i.e. a hyperbolic curve that rises linearly as substrate concentrations increase and then begins to level off and approaches a maximum at higher substrate concentrations. Thus, the maximum velocity ($V_{max}$) and the half-maximal velocity ($K_m$) also referred to as Michaelis-Menten constant can be derived from the Michaelis-Menten plot. The constant ($V_{max}/K_m$) is a measure of how efficiently an enzyme converts a substrate into product (Bisswanger, Perspectives in Science, 1 :41-55 (2014)). Furthermore, enzyme assays suitable for high throughput have been developed with the aim to provide sufficient performance to support the testing of a broad range of samples in a highly reproducible manner (Acker and Auld, Perspectives in Science, 1 :56-73 (2014); Inglese et al, Nat. Chem. Biol., 3:466 (2007); Inglese et al, Nat. Chem. Biol., 3:438 (2007)).

Moreover, a wide range of screening techniques based on affinity assays have been developed to identify or improve the binding reactions of antibodies for their therapeutic, diagnostic, analytical and chromatographic applications and including for example surface-based methods such as biolayer interferometry (BLI) and surface plasmon resonance (SPR) (Nieri et al, Current Clinical Medicine, 16: 753-779 (2009); Rajpal et al, Proc. Natl. Acad. Sci., 102: 8466-8471 (2005); Dubel et al, Trends Biotechnology, 28: 333-339 (2010)). For example, biolayer interferometry is a label-free technology for measuring biomolecular interactions based on an optical analytical technique which detects the interference pattern of white light reflected from two layers comprising a layer of immobilized protein on the biosensor and a reference layer. Changes in the amount of molecules bound to the biosensor result in a shift in the interference pattern which detects the kinetics of association and dissociation of the two molecule molecules as well as the affinity constant for the protein interaction (Fang et al., 2007, Trends in Bio/Pharmaceutical Industry. 3: 34-8; Rich and Myska, 2005, Analytical Biochemistry. 361 (1): 1-6). Another example for a commonly used label-free technique for the measurement of biomolecular interactions is surface plasmon resonance, which measures the change in the refractive index of light reflected from a metal surface (the "biosensor"). In brief, a common application comprises one biomolecule binding to one binding partner that is immobilized on the surface. Upon binding of the biomolecule to the surface, a change in the refractive index is induced which is proportional to the mass added to the surface. Measuring the rates of addition and displacement of one binding partner provided in solution phase to a second binding partner that is immobilized on the surface provides information regarding the affinity of a biomolecule by quantifying dissociation constants ($K_D$) (Hemerhorst et al., 2012, Clin Biochem Rev 33(4): 161-173).

Although production and screening of individual proteins and peptides with high affinities down to the picomolar range is today feasible with state-of-the-art technologies, such technologies usually require expensive equipment and do not lend themselves well to true high throuput analysis. Hence, they are rather inefficient, cost and labor intensive. For example, while SPR-based measurements often yield affinities that agree well with solution methods (Day et al., 2002, Protein Sci 11: 1017-1025; Myszka et al., 2003, J Biomol Tech 14: 247-269; Bee et al., 2012, PLOS ONE 7: e36261; Drake et al., 2004, Anal Biochem 328: 35-43), the use of a surface can sometimes give misleading results due to the introduction of artefacts, which may be influenced by the choice of sensor chip type used (Abdiche et al., 2011, Anal Biochem 411: 139-151) and the relative charges of the analyte/ligand pair being studied (Drake et al., 2012, Anal Biochem 429: 58-69). Further, since affinity measurement by SPR is usually based on measuring kinetic rates, especially the long off rates that are associated with high affinity interactions are often difficult to measure in high throughput.

Due to increasing demands in sensitivity, diverse sample applicability and throughput, the set up of biophysical affinity assays has been transformed significantly over the past decade to implement multiwell measurements and automation modules. Such advancement is needed for improving the efficacy of the research and development of protein-based entities for commercial applications by decreasing the resource requirement and widening the experimental design space. In addition, with the beginning of the development of recombinant proteins for commercial applications, the greater chemical complexity of proteins led to greater sizes of mutant libraries to be tested and has also demanded the need of the use of high throughput biophysical affinity screening assays. The benefits of applying high-throughput technologies in the development of proteins for commercial applications from early stages of candidate screening and selection through to the later stages are obvious in terms of the potential for time and cost saving. Ideally high-throughput assays should also be designed such that they only require small volumes and sample amounts. Saving on the amount of sample required can help overcome the inefficiencies of production and purification processes for providing the samples which at the screening stage may not have been optimized. A third advantage of high throughout assays is that they permit many more drug candidates to be tested within a shorter period of time than would be possible using a standard approach. Recent microfluidic and automation technologies have been used to develop higher-throughput kinetic assays. For example, the Biacore A100 biosensor is based on the SPR principle and can process multiple samples. However, the scope for multiplexing with these technologies is still limited and automated sample loading and running can be hampered for samples that have to be processed in sequence and which have e.g. slow off rates that take hours rather than seconds or minutes to measure.

Hence, although high-throughput biophysical methods become increasingly important in the development of therapeutic proteins, most biophysical methods have not been adapted to true high-throughput formats where hundreds or thousands of samples can be analysed in one to two days.

It is clear that there is a need for new and better high-throughput biophysical assays and that the needs of the biopharmaceutical industry will play a role in their development.

In particular, there is a need of high throughput screening methods for identifying a relevant commercially applicable proteins that can be performed efficiently. This means, that screening of candidates should allow testing of large libraries of recombinant proteins to be screened at low cost, in a short time, whilst still providing high quality data on binding affinities and other activity properties.

For the first step of such a screening method, i.e. the establishment of mutant libraries to be screened, recombinant DNA technology has been widely applied as an important tool in engineering and developing new generation therapeutic proteins and peptides produced by and purified from a wide range of organisms.

As such, traditional strategies for recombinant protein expression are cell-based and involve the combination of an expression vector, that contains the template of the cloned DNA and the host vector that provides a context to allow foreign gene function in a host cell, that is, produce proteins at a high level. The expression vector is delivered into the host cell by transfection of cells with the DNA vector that contains the template and then culturing the cells so that they transcribe and translate the desired protein. Commonly used production systems for recombinant protein expression include host cells derived from bacteria, yeast, baculovirus/insect cells, mammalian cells, and transgenic animals and plants (Mason et al., Trends Mol. Med. 8: 324-329 (2002); Wurm and Bernard, Curr. Opin.Biotechnol. 10: 156-159 (1999); Zoller, Cur. Opin. Biotechnol. 2:526-531); Brannigan and Wilkinson, Nature Rev. Mol. Cell Biol. 3: 964-970 (2002); Watson et al., Recombinant DNA, Scientific American Books, New York, 453-470 (1992)). Commonly used DNA sources and delivery mechanisms are viruses, plasmids, artificial chromosomes and bacteriophage. Typically, the cells are then lysed to extract the expressed protein for subsequent purification, or the protein is recovered from cell culture supernatant. The selection of the system depends on the type of protein, the requirements for functional activity and the desired yield. Bacterial protein expression systems are popular because bacteria are easy to culture, grow fast and produce high yields of recombinant protein. However, multi-domain eukaryotic proteins expressed in bacteria often are non-functional because bacteria are prokaryotes and are not equipped with the full enzymatic machinery to accomplish the required post-translational modifications or molecular folding. Furthermore, many proteins become insoluble as inclusion bodies that are very difficult to recover without harsh denaturants and subsequent cumbersome protein-refolding. To address these concerns, expression systems using eukaryotic mammalian cells were developed as these cells usually produce fully folded and thus functional protein. Unless expression is optimized, mammalian expression systems can have low yield, high costs of production and be rather time-consuming. In addition, such *in vitro* systems are not conducive to either high throughput protein synthesis or expression of proteins that are toxic to host cells.

Cell-free protein expression system, also referred to as *in vitro* translation, IVT, or cell-free expression is the *in vitro* synthesis of protein using translation-compatible extracts of whole cells. Common components of a cell-free translation system include a cell extract from rabbit reticulocytes, wheat germ and *E. coli* (Carlson et al., 2012, Biotechnol. Adv. 30 (5): 1185-94). The extracts are prepared as crude extracts containing all the required macromolecular components for translation of exogeneous RNA including 70S or 80S ribosomes, tRNAs, aminoacyl-tRNA synthetases, translation initiation and elongation factors, nucleases, etc.. To ensure efficient translation, each extract must be supplemented with energy sources (ATP, GTP), amino acids, co-factors and the starting material consisting of RNA or DNA with the desired genes. Cell-free protein expression systems have several advantages over traditional cell-based *in vitro* systems for protein production such as bacterial fermentation and cell culture. Most notably, cell-free expression allows for fast synthesis of proteins within a few hours without the requirement of gene transfection and cell culture, whereas cell-based expression systems typically take days or weeks to run from start to finish.

Moreover, IVT is essentially free from many cellular mechanisms that regulate gene expression except protein production itself. IVT protein synthesis therefore has advantages in the production of cytotoxic, and otherwise difficult to express proteins. In addition, IVT assays allow direct access and manipulation of the endogenous components by chemicals, enzymes, or modifications by the addition of recombinant proteins due to the absence of cell wall (Ohlmann et al. EMBO J., 16(4): 844-855, 1997; Ohlmann et al. J Mol Biol., 318(1): 9-20, 2002; Ziegler et al. Virology, 213(2): 549-557, 1995; Ziegler et al. J Virol., 69(6): 3465-3474, 1995). Hence, cell-free systems allow the rapid expression of many different proteins simultaneously and thus allow testing protein mutations by expression on a small scale from many different recombinant DNA templates. Thus, IVT is routinely exploited to perform high throughput production of protein libraries (Goshima et al. Nat Methods, 5(12): 1011-1017, 2008). As IVT has been an important tool in many areas of bioengineering, molecular biology, diagnostic assays, and biological discovery, further applications of IVT include rapid identification of gene products (e.g., proteomics), localization of mutations through synthesis of truncated gene products, protein folding studies, and incorporation of modified or unnatural amino acids for functional studies (Lu et al., 2017, Synthetic and Systems Biotechnology, 2(1): 23-27). Hence, the utility of IVT is that it can save significant amounts of time in obtaining an expressed protein, which, in turn, is uselful in generating a mutant library of polypeptides, parallel protein synthesis, and optimization of expression constructs, among others.

However, the generation of therapeutic protein candidates with desired affinity by combination of constructing such mutant libraries with sophisticated affinity strategies using methods known in the art suffer from several drawbacks. In particular, only low yields can be obtained by IVT compared to *in vivo* expression systems. Moreover, crude expressed proteins obtained by IVT typically contain too much matrix contamination to work well in affinity assays such as SPR and biolayer interferometry without further purification due to the high background noise produced by the contam inants.

Combining the generation of mutant libraries by IVT and a method for determining the affinity of a polypeptide and/or the activity of an enzyme, or the activity of an enzyme by its interaction with variants of a substrate of such enzyme, or with variants of a regulatory of such enzyme, which can be performed in high throughput mode has to be carefully adapted. With respect to high-throughput screening (HTS) enzyme assays, it is important to determine the enzyme activity, e.g. by measuring the enzyme velocity at a range of substrate and/or where applicable regulatory protein or co-factor concentrations. In this context, e.g. the enzyme reaction of multiple batches with fixed enzyme concentration and varying substrate concentrations can be compared. However, due to the divergent features of enzymes, many parameters need to be considered for developing robust and sensitive enzyme assays suitable for HTS screening, such as buffer and salt concentrations, pH, co-factor requirements, temperature, concentrations of substrates, enzymes and additives. With respect to the screening of large libraries of mutants of enzymes, mutants of substrates of enzymes, mutants of regulatory proteins of enzymes, for the development of molecules with improved activity for commercial applications, e.g. therapeutic application, there is a need for constructing robust and sensitive enzyme assays as HTS. Although recent advances in the development of HTS enzyme assays have been achieved, further improvement of HTS enzyme assays is required in particular for providing efficient and cost-effective procedures.

With respect to HTS ligand binding assays, essential features for implementation into a HTS assay are (1) estimation of the degree of affinity by the equilibrium dissociation constant ($K_D$) of the respective clones; (2) reliable identification of affinity-improved candidates; (3) high-throughput processing by automation of all sample-handling steps; and (4) compatibility with crude production matrices, such as bacterial extracts or cell culture supernatants. Conventional binding assays for affinity determination well known in the art including surface plasmon resonance (SPR; e.g., Biacore) and biolayer interferometry (BLI; e.g., Octet) only partially fulfill these criteria. For example, in view of the affinity, it is a major difficulty to estimate the $K_D$ in the picomolar (pM) range with a reasonable throughput. This problem has been addressed by Della Ducata et al. (Journal of Biomolecular Screening, 20(10): 1256-1267, 2015) describing a high-throughput screening (HTS) method based on the principle of solution equilibrium titration (SET) immunoassay (see for example Friguet B et al. (Protein Structure, A Practical Approach, Second Ed. 1997, Chapter 13, Section 5, p.335) for an introduction) using electrochemiluminescence (ECL) as a readout system for affinity estimation of antibodies derived from phage display libraries in crude sample matrices. Different purification steps can be applied to improve the specificity of the affinity assay, but make the method more labor intensive.

Many desired mutant libraries are typically very large, i.e. 100s to 1000s of variants are intended to be screened, so that screening using affinity strategies are impractical with current technologies, inefficient and labor intensive. In view of these drawbacks, the overall generation of proteins for commercial applications using a combination of expression system technology and affinity/activity strategies is often time-consuming due to the need of cell culture and protein purification as well as unsatisfactory efficiency due to large-scale libraries, impurity of the sample, low yield and/or concentration of sample that is not compatible to rapidly and efficiently estimate affinities.

Thus, there is a need for screening methods enabling the screening of a large amount of individual polypeptide variants simultaneously, by providing less time-consuming techniques, in particular less time-consuming recombinant protein expression technologies, while maintaining or more preferably improving the specificity of high throughput affinity assays and keeping cost-effective procedures of the HTS screening. The present invention is directed toward satisfying such needs by providing a method for screening the activity of a polypeptide and for producing a polypeptide with a desired activity in an economic and efficient manner with high specificity, which can be performed in a high-throughput mode.

Relevant prior art citations:

Huber (2007); J. Struct. Biol. 159:206-221
WO201705909
Yin (2012); mAbs 4(2):217-225

## Summary of the Invention

The above drawbacks and disadvantages of the prior art are overcome by the present invention, which relates to the embodiments as characterized in the claims. Thus, in a first aspect, the present invention provides a method for screening the activity of a polypeptide comprising (i) expressing a polypeptide by coupled cell-free *in vitro* transcription-translation (IVT) and (ii) determining the activity of the expressed polypeptide by solution equilibrium titration with an interaction member, wherein the activity is the binding affinity of the polypeptide to the interaction member.

Preferably, the method is for producing a polypeptide with a desired activity.

In a preferred embodiment, the methods of the present invention comprise introducing at least one point mutation in the amino acid sequence of the polypeptide to be expressed in step (i). Preferably, the said at least one point mutation is generated by site-directed mutagenesis. More preferably, the said at least one point mutation comprises one or more amino acid substitutions, deletions, additions, and/or insertions.

In a preferred embodiment, the method comprises the expression and screening of at least 100 polypeptide variants.

In a preferred embodiment, the $K_D$ of the binding affinity is less than 1 nM, preferably less than 100 pM and more preferably less than 10 pM.

In a preferred embodiment, the polypeptide is selected from the group consisting of antibodies and fragments thereof, receptors, preferably T cell receptors, enzymes, substrates of enzymes, regulatory proteins of enzymes, cytokines, Fc fusion proteins, anticoagulants, blood factors, bone morphogenetic proteins, engineered protein scaffolds, growth factors, hormones, interferons, interleukins, and thrombolytics.

In a preferred embodiment, the expressed polypeptide or its interaction member contains a binding domain of an antibody fragment.

In a preferred embodiment, the expressed polypeptide or its interaction member contains the active domain of an enzyme, or the active domain of a substrate of an enzyme, or the active domain of a regulatory protein of an enzyme.

In a preferred embodiment, the screening comprises identifying a polypeptide with an affinity to an interaction member that is altered as a consequence of one or more introduced point mutations in said polypeptide.

In a preferred embodiment, the screening comprises identifying a polypeptide with an affinity to an interaction member that is not substantially altered as a consequence of one or more introduced point mutations in said polypeptide.

In a preferred embodiment, the screening comprises identifying a polypeptide with an affinity to an interaction member that is either increased or not substantially altered as a consequence of one or more introduced point mutations and wherein one or more of said point mutations reduces the binding of a sub-sequence of the polypeptide comprising at least one of said mutations to an MHC molecule.

In a preferred embodiment, the screening comprises identifying a polypeptide with a cross-reactivity between more than one interaction member, and wherein the extent of such cross-reactivity is altered as a consequence of one or more introduced point mutations in said polypeptide.

In a preferred embodiment, the polypeptide to be screened is an enzyme, or a substrate of an enzyme or a regulatory protein of an enzyme, and the activity of the polypeptide is the activity of the polypeptide in a corresponding enzyme reaction.

Preferably, the increased half-life *in vivo* as a consequence of one or more introduced point mutations in the polypeptide(s) is due to an altered binding affinity of said polypeptide(s) to FcRn.

**Brief Description of the Drawings**

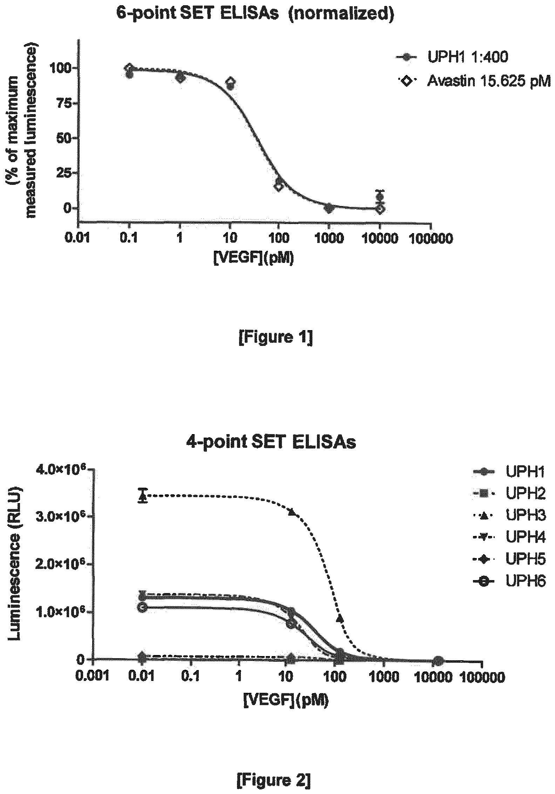

Figure 1: Free Avastin or wild type Avastin single chain variable fragment (wt Av scFv) present in equilibrium with varying concentrations of VEGF as measured by SET ELISA. Curves have been normalised to maximum (100 %) and minimum (0 %) luminescence signals. Data shown are of one experiment performed in duplicate.

Figure 2: Free UPH1-6 present in equilibrium with varying concentrations of VEGF as determined by SET ELISA. Data shown are of one experiment performed in duplicate.

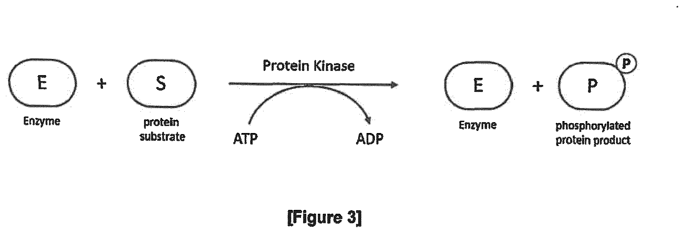

Figure 3: The biochemical reaction catalyzed by a protein kinase. A phosphate group (P) is transferred from a molecule of ATP to a phosphoacceptor protein to produce a phosphoprotein and a molecule of ADP.

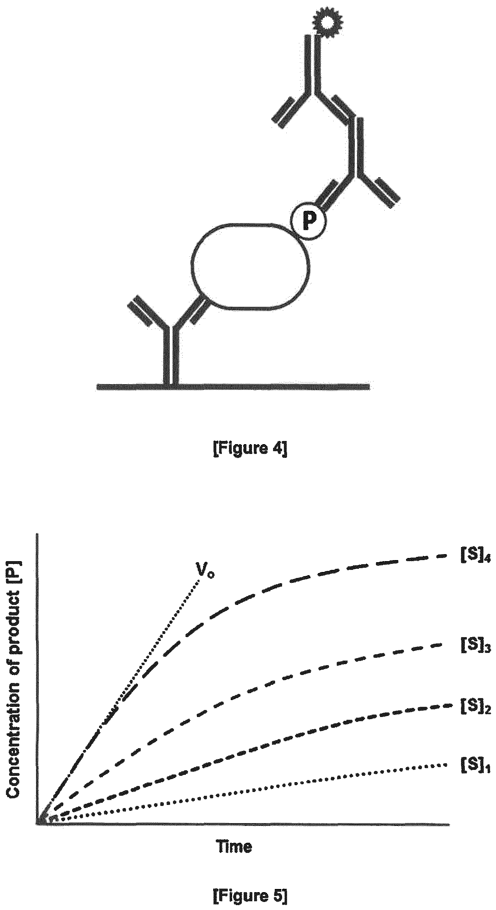

Figure 4: Schematic representation of a sandwich ELISA-based assay used as discontinuous assay for determination of the reaction velocity (Va). The product of an enzyme reaction (phosphoprotein formed by the catalytic reaction of a protein kinase) is immobilized on a surface coated with an antibody directed against the product. A secondary antibody binds to the phosphate group of the phosphoprotein. A detection antibody linked with a detection marker is directed against the Fc portion of the secondary antibody.

Figure 5: Measurement of concentration of product [P] over time at a fixed enzyme concentration and different substrate concentrations ($[S]_1$, $[S]_2$, $[S]_3$, $[S]_4$) for the determination of the reaction velocity (Va). The reaction velocity is determined from the slope of the linear part of the progress curve as exemplarily shown for progress curve $[S]_4$.

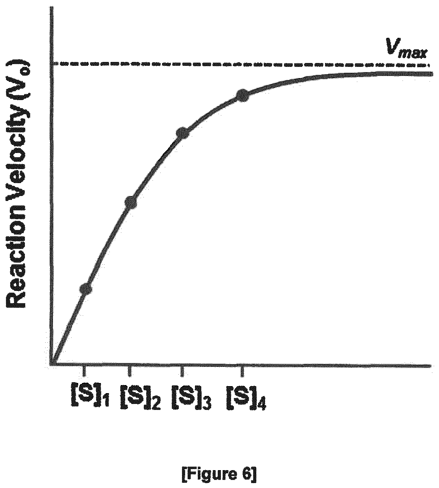

Figure 6: Michaelis-Menten plot of reaction velocity (Va) as a function of substrate concentration [S]. Values for reaction velocity ($V_0$) were obtained from continuous measurement of concentration of product [P] over time at a fixed enzyme concentration and different substrate concentrations ($[S]i$, $[S]_2$, $[S]_3$, $[S]_4$) as provided by Figure 5.

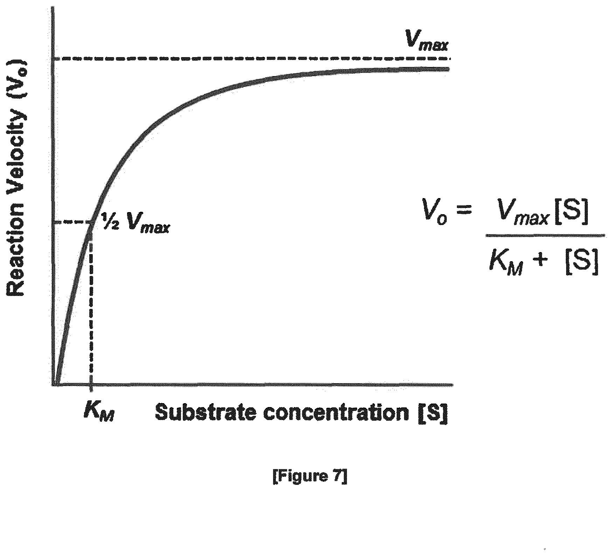

Figure 7: Michaelis-Menten plot of reaction velocity (Va) as a function of substrate concentration [S]. $V_{max}$ represents the maximum reaction velocity achieved by the system at saturating substrate concentrations [S], **and the** Michaelis-Menten constant, $K_M$, is the substrate concentration at which the reaction rate is half of $V_{max}$. The Michaelis-Menten equation is shown to the right.

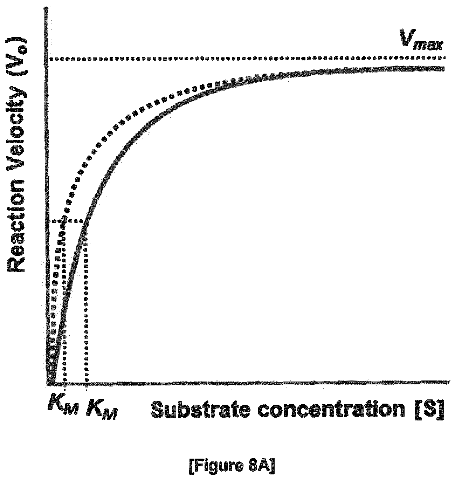

Figure 8A: Michaelis-Menten curves illustrating the kinetic properties of enzymes with improved efficiencies relative to the wild-type enzyme.

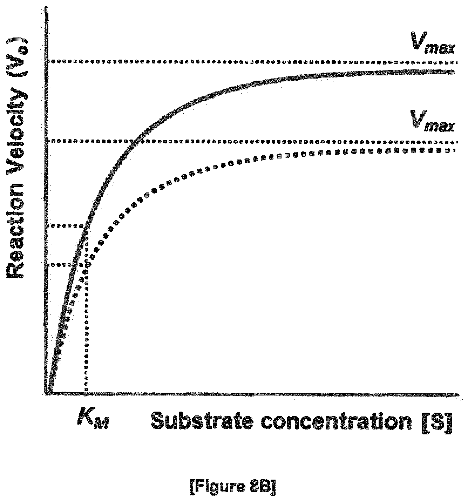

Figure 8B: Michaelis-Menten curves illustrating the kinetic properties of enzymes with decreased efficiencies relative to the wild-type enzyme.

**Detailed Description of the Invention**

The technical disclosure set out below may in some respects go beyond the scope of the claims. Elements of the disclosure which do not fall within the scope of the claims are provided for information. $

The present invention provides a method that overcomes the above-mentioned drawbacks and disadvantages of the prior art. Accordingly, the present invention relates to a method for screening the activity of a polypeptide comprising (i) expressing a polypeptide by coupled cell-free *in vitro* transcription-translation (IVT) and (ii) determining the activity of the expressed polypeptide by solution equilibrium titration with an interaction member, wherein the activity is the binding affinity of the polypeptide to the interaction member.

Preferably, the method is for producing a polypeptide with a desired activity.

The present invention is based on the finding that, by applying the aforementioned methods, low amounts of and less purified polypeptides can be screened for a desired activity in an efficient, i.e. high throughput setting, and cost-effective manner. Thereby, the affinity and/or activity of the polypeptide can be determined with high specificity. The methods of the invention are particularly useful in connection with the development of a therapeutic polypeptide, typically involving the screening of a large library of polypeptides requiring techniques capable to be performed in high-throughput formats with high specificity while at the same time being cost-effective and less time-consuming. The methods of the invention are particularly useful for screening of an end-phase engineered polypeptide in order to further optimize a commercial protein agent in the final stages of design with respect to one or more remaining functional features (such as the removal of immunogenic epitopes while maintaining good binding properties, removal of undesired amino acids, changing the charge surface of the protein, changing the cross-reactivity between said protein and two or more binding partners, changing stability, increasing half life, improving expression yields, and improving ease of purification).

The singular form "a", "an" and "the" include plural referents unless the context clearly dictates otherwise. Thus, for example, reference to "a polypeptide" includes a plurality of polypeptides. The terms "comprising," "having," "including," and "containing" of the present invention are to be construed as open-ended terms (i.e., meaning "including, but not limited to") unless otherwise noted. Recitation of ranges of values herein are merely intended to serve as a shorthand method of referring individually to each separate value falling within the range, and includes the endpoint boundaries defining the range, unless otherwise indicated herein, and each separate value is incorporated into the specification as if it were individually recited herein.

In general, the terms and phrases used in accordance with the present invention are to be construed with ordinary and typical meaning to those of ordinary skill in the art, and can be found by reference to standard texts, journal references and contexts known to those skilled in the art. The following definitions are provided to clarify their specific use in the context of the present invention.

The term "polypeptide" used in accordance with the present invention refers to a sequence of covalently linked amino acids of any length and is intended to be used with the terms "peptide," "oligopeptide," or "protein" interchangeably. Polypeptides of the present invention refer to a chain of two or more amino acids which are linked together with peptide or amide bonds. In accordance with the present invention, polypeptides can comprise more than one subunit, where each subunit is encoded by a separate DNA sequence. As used in the present invention, the screening of polypeptides includes wild-type polypeptides and mutant polypeptides. The terms "wild-type polypeptide", "wt" and "reference polypeptide" are used interchangeably in accordance with the present invention and refer to a polypeptide having an amino acid sequence isolated from a naturally occurring or non-modified polypeptide, e.g., the original or parent polypeptide clone, with specificity for a known interaction member. As used in accordance with the present invention, the terms "modified", "variant", "mutant", "mutated" and "derived" refer to a variant of polypeptide that has one or more mutations in the amino acid sequence of the wild-type polypeptide. In a preferred embodiment of the invention, the polypeptide comprises introducing at least one point mutation in the amino acid sequence of the polypeptide to be expressed by coupled cell-free *in vitro* transcription-translation (IVT). As used in accordance with the present invention, the term "point mutation" refers to a change of the nucleotide present at a site of a mutation in the mutant copy of a genomic locus causing a change in the encoded amino acid sequence. The nucleotide may be on either strand of a double stranded DNA molecule. In accordance with the present invention, the point mutation comprises insertions, deletions, substitutions and additions in the DNA or RNA encoding the polypeptide. The term "genomic locus" of the present invention refers to a defined region in a genome. As provided in the methods of the present invention, the term "amino acid sequence" refers to a contiguous sequence of amino acids.

In the context of the present invention, the term "at least one point mutation" refers to the substitution, deletion, addition or insertion of one to 25 amino acid residues in the polypeptide sequence, preferably, one to 5 amino acid residues in the polypeptide sequence, and more preferably one amino acid residues in the polypeptide sequence.

In a preferred embodiment, the said at least one point mutation is generated by site-directed mutagenesis. As provided in the methods of the present invention, the term "site-directed mutagenesis" refers to any suitable known method in the art to introduce one or more point mutations in the amino acid sequence of the polypeptide. For example, one or more point mutations can be introduced by the use of an oligonucleotide encoding the desired mutation which is annealed to one strand of a DNA of interest and serves as a primer for initiation of DNA synthesis. In this manner, the oligonucleotide primer may be incorporated into the newly synthesized strand. Methods for generating site-directed mutagenesis in accordance with the methods of the present invention are well known in the art (Angag and Schutz, Biotech. 30: 486-488 (2001); Wang and Wilkinson, Biotech. 29: 976-978 (2000); Kang et al., Biotech.20: 44-46 (1996); Ogel and McPherson, Protein Engineer. 5: 467-468 (1992); Kirsch and Joly, Nuc. Acids. Res. 26: 1848-1850 (1998); Rhem and Hancock, J. Bacteriol. 178: 3346-3349 (1996); Boles and Miogsa, Curr. Genet. 28: 197-198 (1995); Barrenttino et al., Nuc. Acids. Res. 22: 541-542 (1993); Tessier and Thomas, Meths. Molec. Biol. 57: 229-237; and Pons et al., Meth. Molec. Biol. 67: 209-218). Briefly, the key elements of the method include introducing a mutation into the DNA sequence of a polypeptide using a single oligonucleotide mutagenic primer, which in an extension/polymerization reaction, produces one or more newly synthesized copies of the DNA sequence comprising the primer with the mutation. Preferably, the reaction is cyclic which produces a multiplicity of the mutated copies of the DNA. A selective means is used for degrading or removing the wild-type DNA from the newly synthesized copies of the DNA with the mutation and propagation of the copies in host cells. Thus, the method comprises three steps (1) design and synthesis of the primer, (2) annealing the primer to a parental target DNA and producing one or more mutated copies of the parental target DNA in a single reaction, and (3) degrading or removing the parental target DNA and propagating the mutated copies in host cells. In a preferred embodiment of the invention, the polypeptide comprising at least one mutation further comprises one or more amino acid substitutions, deletions, additions, and/or insertions compared to the wild-type polypeptide clone. A "substitution", as used in accordance with the present invention, refers to the replacement of one or more amino acids or nucleotides by different amino acids or nucleotides, respectively. A "deletion", as used in the present invention, refers to a change in the amino acid or nucleotide sequence that results in the absence of one or more amino acid residues or nucleotides. The terms "addition" and "insertion" are used interchangeably in the present invention and refer to a change in the amino acid or nucleotide sequence that result in the presence of one or more additional amino acid residues or nucleotides. As used in the methods of the present invention, identification of a point mutation in the amino acid sequence of a polypeptide to be expressed in step (i) can be performed by methods well known in the art and include but are not limited to Sanger Sequencing or the more recent next generation sequencing technology (Sanger and Coulson, J. Mol. Biol., 94(3):41-448 (1975); Sanger et al., Proc. Natl Acad. Sci, 74(12):5463-5467 (1977); Mardis, Annu. Rev. Genomics Hum. Genet., 9:387-402 (2008); Metzker, Nat. Rev. Genet., 11:31-46 (2010); Shendure and Ji, Nat. Biotechnol., 26:1135-1145 (2008)). Next generation sequencing allows faster and cheaper sequencing of DNA and RNA compared to the previously used Sanger sequencing. A number of different next generation sequence technologies include but are not limited to Illumina (Solexa) sequencing, Roche 454 sequencing or Ion torrent sequencing (Kulski et al., Intech, p.141-481 (2014); Aird et al, Kozareva et al., Nat. Methods, 6:291-295 (2009)). Further methods well known in the art include but are not limited to quantitative PCR using TaqMan® (Lie et al., Curr Opin Biotechnol, 9: 43-8 (1998)); quantitative assay using molecular beacons (Tan et al., Chemistry 6: 1107-11 (2000)); fluorescence in situ hybridization FISH (Laan et al., Hum Genet 96:275-80 (1995)) or comparative genomic hybridization (CGH) (Forozan et al., Trends Genet, 13:405-9 (1997)); isothermic DNA amplification (Lizardi et al., Nat Genet, 19:225-32 (1998)); and quantitative hybridization to an immobilized probe(s) (Southern, J. Mol. Biol. 98: 503 (1998)).

In a preferred embodiment of the invention, the polypeptide to be expressed is selected from the group consisting of antibodies and fragments thereof, receptors, preferably T cell receptors, enzymes, substrates of enzymes, regulatory proteins of enzymes, cytokines, Fc fusion proteins, anticoagulants, blood factors, bone morphogenetic proteins, engineered protein scaffolds, growth factors, hormones, interferons, interleukins, and thrombolytics. In a particular preferred embodiment, the polypeptide is selected from the group consisting of antibodies and fragments thereof, receptors, preferably T cell receptors, enzymes and Fc fusion proteins. More preferably, the polypeptide is selected from the group consisting of antibodies and fragments thereof and T cell receptors. Most preferably, the polypeptide is an antibody or fragment thereof. In a further embodiment of the present invention, the polypeptide is an enzyme, or a substrate of an enzyme, or a regulatory protein of an enzyme. It is to be understood that the above-mentioned polypeptides include both the wild-type and the mutant polypeptides selected from said group.

In connection with the present invention, the term "antibody" is used herein in accordance with the art-establishing meaning and refers to all types of immunoglobulins and mutants thereof which are capable of specifically binding to an antigen. As used in the present invention, antibodies may comprise one, or several units, each of which consisting of two heavy (H) polypeptide chains and two light (L) polypeptide chains. Generally, the H and L chains are made up of a series of domains. The L chains, of which there are two major types ($\kappa$ and $\lambda$), consist of two domains. The H chains are of several types, including $\mu$, $\delta$, and $\gamma$ (of which there are several subclasses), $\alpha$ and $\epsilon$. In humans, there are eight genetically and structurally identified antibody classes and subclasses as defined by heavy chain isotypes: IgM, IgD, IgG1, IgG2a, IgG2, IgG3, IgG4, IgE, and IgA. Further, for example, "IgG" means an antibody of the G class, and "IgG1" refers to an IgG molecules of subclass 1 of the G class. A constant region in the antibodies used in connection with the present invention is not particularly limited, and any constant region may be used. Preferred examples of the constant region include human antibody-derived constant regions (for example, C$\gamma$1, C$\gamma$2, C$\gamma$3, C$\gamma$4, C$\mu$, C$\delta$, C$\alpha$1, C$\alpha$2, and C$\epsilon$ for the H chain, and C$\kappa$, C$\lambda$, and such for the L chain). Particularly preferred examples of natural human antibody constant regions include constant regions derived from IgG1, IgG2a, IgG2b, or IgG4.

Furthermore, as noted above, the immunoglobulin (antibody), or fragment thereof used in the present invention may be monoclonal in nature. The genetic code of monoclonal antibodies used for IVT of the present invention can be derived from the genetic code of such monoclonal antibodies in hybridomas which are publicly available from sources such as the American Type Culture Collection ("ATCC") 10801 University Boulevard, Manassas, Va. 20110-2209.

In accordance with the present invention, the immunoglobulin can for example be monoclonal, chimeric, bifunctional and hybrid antibodies or fragment thereof. The terms "chimeric", "bifunctional" and "hybrid" have their general meaning in the art.

For example, a chimeric antibody or fragment thereof is a monoclonal antibody comprising a variable region, i.e. binding region, from one source or species and at least a portion of a constant region derived from a different source or species, usually prepared by recombinant DNA techniques. Chimeric antibodies and fragment thereof comprising a non-human variable region, for example from mouse or monkey, and a human constant region are preferred in certain applications of the invention, particularly human therapy, because such antibodies are readily prepared and may be less immunogenic than purely non-human monoclonal antibodies. Methods for producing chimeric antibodies are well known in the art, e.g., Morrison et al., Proc. Nat'l Acad. Sci. 81:6851 (1984). The term "humanized" refers to those antibodies and fragment thereof in which one or more of the "complementarity" determining regions ("CDR") from a non-human immunoglobulin molecule have been introduced to substantially replace the CDRs of an immunoglobulin molecule with human framework regions. Common genetic engineering techniques for producing humanized antibodies are known in the art (see EP Patent Application Publication No. EP 125023 and WO 96/02576). A humanized antibody is obtained by linking the obtained DNA to a DNA encoding a human antibody constant region or a modified human antibody constant region, then incorporating this into an expression vector, and transfecting the vector into a host to produce antibodies (see EP Patent Application Publication No. EP 239400 and WO 96/02576).

In addition to approaches wherein antibodies may be converted to increasingly more human-like antibodies, techniques to produce fully human monoclonal antibodies and fragments thereof have been developed in which monoclonal antibodies are in fact devoid of any non-human sequences. Such techniques are generally known in the art and include, for example, employing human antibody genes in phage display libraries or using human antibody genes in transgenic mice (Hoogenboom et al., Immunol Today, 21(8):371-8 (2000); Gavilondo et al., Biotechniques, 29(1): 128-32 (2000)). In the human antibody gene phage display technique, genes encoding the VH and VL chains are generated by polymerase chain reaction (PCR) cloning from "naive" human lymphocytes; these genes are then assembled into a library from which they can be expressed either as disulfide-linked Fab fragments or as single-chain Fv (scFv) fragments; the Fab- or scFv-encoding genes are also fused to a surface coat protein of filamentous bacteriophage (Iba, Immunol. Cell Biol. 75, 217-221 (1997); Marks, N. Engl. J. Med. 335, 730-733 (1996)). Preferably, human antibodies are used in accordance with the present invention.

The bifunctional or hybrid antibody or fragment thereof refers to an antibody in accordance with the present invention that comprises within the same antibody molecule, variable regions recognizing different epitopes. For example, the bifunctional antibody may be one in which each arm has specificity for a different epitope such as of a tumor associated antigen of the cell to be therapeutically or biologically modified. In any case, the hybrid antibody has a dual specificity, preferably with one or more binding sites specific for an antigen, for example, an antigen associated with a tumor, an infectious organism, or other disease state. Bispecific antibodies can be prepared with genetic engineering techniques known in the art.

In any event, the present invention should not be construed as limited in scope by any particular method of production of an antibody whether bifunctional, chimeric, humanized, human or an antigen-recognizing fragment or derivative thereof.

In connection with the present invention, the term "antibody fragment" refers to molecules which specifically bind to an antigen and which are derived from a whole immunoglobulin by cleavage, by recombinant methods or by any other process that results in a functional equivalent of a conventional antibody fragment. Preferably, antibody fragments are not particularly limited as long as they can bind to an interaction member. The antibody fragments used in the present invention are not particularly limited so long as they include a part of a whole antibody, but preferably include either VH or VL, and particularly preferably include both VH and VL. Another preferred example of the antibody fragments used in the present invention includes fragments comprising antibody CDRs. The CDRs included in the antibody fragments may be all six CDRs of the antibody or some of the CDRs. Such antibody fragments, also referred to as minibodies typically have a smaller molecular weight compared to the whole antibody; however, they may form multimers such as a dimer, trimer, or tetramer, and their molecular weight may become larger than the whole antibody.

As one skilled in the art will recognize, such fragments can be prepared with recombinant techniques using DNA sequences of the immunoglobulin fragment. Recombinant DNA techniques to produce antibody fragments comprise constructing genes encoding the antibody fragments, introducing the gene into an expression vector, and then expressing these antibody fragments (see, for example, Co, M.S. et al., J. Immunol. (1994) 152, 2968-2976; Better, M. and Horwitz, A.H., Methods in Enzymology (1989) 178, 476-496; Plueckthun, A. and Skerra, A., Methods in Enzymology (1989) 178, 476-496; Lamoyi, E., Methods in Enzymology (1989) 121, 652-663; Rousseaux, J. et al., Methods in Enzymology (1989) 121, 663-669; and Bird, R.E. et al., TIBTECH (1991) 9, 132-137).

Examples of antibody fragments include Fab, Fab', F(ab')2, Fv, scFv (single-chain Fv), diabody, sc(Fv)2 (single-chain (Fv)2), etc. Polymers thereof (such as dimers, trimers, tetramers, or polymers) are also included in the antibody fragments used in the present invention. In a preferred embodiment of the invention, the immunoglobulin may be a single chain antibody ("SCA"). These may consist of single chain Fv fragments ("scFv"). A scFv is an antibody of a single-chain polypeptide obtained by linking VH and VL through a linker or such (Huston, J.S. et al., Proc. Natl. Acad. Sci. U. S. A. (1988) 85, 5879-5883; and Plueckthun, "The Pharmacology of Monoclonal Antibodies" Vol.113, Ed Resenburg and Moore, Springer Verlag, New York, pp.269-315, (1994)). The H chain V region and L chain V region in a scFv may be derived from any antibody described herein. There is no particular limitation on the peptide linkers that link the V regions. The amino acid sequence of the peptide linker can be suitably selected by those skilled in the art according to the objective. For example, the variable light ("V[L]") and variable heavy ("V[H]") domains are linked by a peptide bridge or by disulfide bonds.

The V regions of both chains linked by a peptide bridge can be produced by PCR methods known in the art. To link the V regions using the PCR method, a DNA encoding the entire or desired partial amino acid sequence of the DNAs comprising a DNA sequence encoding the H chain or the H chain V region of the antibody, and a DNA sequence encoding the L chain or the L chain V region of the antibody is used as template. Each DNA encoding the V regions of the H chain or L chain is amplified by the PCR method using pairs of primers with sequences corresponding to the sequences at both ends of the DNA to be amplified. Next, a DNA encoding the peptide linker portion is prepared. The DNA encoding the peptide linker can also be synthesized by PCR. Nucleotide sequences that can link the amplification products of each separately synthesized V region are added to the 5'side of the primers used at this time. Next, a PCR reaction is carried out using the "H chain V region DNA", the "peptide linker DNA", and the "L chain V region DNA" together with the primers for the assembly PCR. The primers for the assembly PCR consist of a combination of a primer that anneals to the 5' side of the "H chain V region DNA" and a primer that anneals to the 3'side of the "L chain V region DNA". Therefore, the primers for the assembly PCR consist of a primer set that can amplify the DNA encoding the entire sequence of the scFv to be synthesized. Conversely, nucleotide sequences that can link to each V region DNA are added to the "peptide linker DNA". As a result, these DNAs are linked together and the full-length scFv is finally produced as an amplification product of the primers used for the assembly PCR. The order of VHs and VLs to be linked is not particularly limited, and they may be arranged in any order, for example as [VH] linker [VL] or as [VL] linker [VH]. In a particularly preferred embodiment, the scFv may be Avastin scFv, as also illustrated in the Examples of the present invention.

The term "receptor" as used in accordance with the present invention refers to the entire receptor or to portions thereof capable of binding to an interaction member, such as the natural ligand of the receptor. The term "T-cell receptor" (TCR) has its general meaning in the art and, as used in connection with the present invention, refers to a molecule capable of recognizing a peptide comprising a T cell epitope when presented by an MHC molecule. The molecule may be a heterodimer of two chains $\alpha$ and $\beta$ (or optionally $\gamma$ and $\delta$) or it may be a single chain TCR construct. In a preferred embodiment of the invention, the receptor is a T cell receptor.

The term "enzyme" has its conventional meaning in the art and, as used herein, encompasses proteins that catalyze at least one biochemical reaction. A compound to which a particular enzyme catalyzes a reaction is typically referred to as a substrate of the enzyme. Generally, enzymes include but are not limited to those catalyzing reduction/oxidation or redox reactions, hydrolysis, removal from or addition to a substrate of specific chemical groups, isomeration, or the combination or binding together of substrate units.

The term "cytokine" has its conventional meaning in the art and, as used in the present invention, particularly refers to a polypeptide that is naturally secreted by mammalian cells and that binds to a cell surface receptor on a leukocyte affecting the functions of other immune cells, and is a molecule which modulates interactions between cells in the immune or inflammatory response. The term "cytokine" also encompasses polypeptides that act as ligand to a receptor for a naturally occurring cytokine. Cytokines as used in the present invention include, but are not limited to, monokines and lymphokines regardless of which cells produce them. For instance, a monokine is generally referred to as being produced and secreted by a mononuclear cell, such as a macrophage and/or monocyte but many other cells produce monokines, such as natural killer cells, fibroblasts, basophils, neutrophils, endothelial cells, brain astrocytes, bone marrow stromal cells, epideral keratinocytes, and $\beta$-lymphocytes. Lymphokines are generally referred to as being produced by lymphocytes. Examples of cytokines include, but are not limited to, interleukin-1 (IL-1), tumor necrosis factor-alpha (TNF$\alpha$) and tumor necrosis factor beta (TNF$\beta$).

The term "growth factor" has the generally accepted meaning in the art. As used in the methods of the present invention, the growth factor refers to a polypeptide that is capable of effecting proliferation and/or differentiation of cells. Examples of growth factors as contemplated for use in accordance with the present invention include but are not limited to epidermal growth factors (EGF), transforming growth factor-alpha (TGF$\alpha$), transforming growth factor-beta (TGF$\beta$), human endothelial cell growth factor (ECGF), granulocyte macrophage colony stimulating factor (GM-CSF), bone morphogenetic protein (BMP), nerve growth factor (NGF), vascular endothelial growth factor (NEGF), fibroblast growth factor (FGF), insulin-like growth factor (IGF), cartilage derived morphogenetic protein (CDMP), and platelet derived growth factor (PDGF).

The term "hormone" has the generally accepted meaning in the art. In accordance with the present invention, it refers to a polypeptide released from a cell into the extracellular fluid in low quantities, which acts on a target cell to produce a response. Hormones comprise endocrine hormones, which are released directly into the bloodstream, and exocrine hormones, which are secreted directly into a duct, and, from the duct, they flow either into the bloodstream or from cell to cell by diffusion in a process known as paracrine signaling. In particular, hormones can comprise polypeptide hormones, lipid and phospholipid-derived hormones and monoamines. For the purpose of the present invention, hormones will be polypeptide hormones. Examples of polypeptide hormones in accordance with the present invention, but not limiting, include insulin and growth hormone.

The term "interferon" has its generally accepted meaning in the art. In accordance with the present invention, it generally refers to a family of polypeptides that inhibit viral replication and cellular proliferation and modulate immune response and include type I interferons (e.g., interferon-$\alpha$ and interferon-$\beta$) as well as type II interferons (e.g., interferon-$\gamma$).

The term "interleukin" has its generally accepted meaning in the art. In accordance with the present invention, it refers to a group of cytokines that are produced by macrophage, lymphocyte, monocyte and endothelial cells. For example, interleukin-1 is produced by the macrophage, and interleukin -2 and interleukin -3 is produced by the lymphocyte.

The term "anticoagulant" has its generally accepted meaning in the art. In accordance with the present invention, it generally refers to any polypeptide having anticoagulant activity. In particular, anticoagulants refer to any polypeptide that interfere, directly or indirectly, with the proteolytic enzymes involved in blood coagulation of vertebrates, preferably mammals and, in particular, humans. For example, the anticoagulants of the present invention include but are not limited to hirudin, antithrombin III, venom and mutants thereof.

The term "blood factors" has its generally accepted meaning in accordance with the present invention and refers to polypeptides, which are involved in the modulation, localization or dissolution of blood clots. For example blood factors of the present invention include but are not limited to Factors II, V, VII, VIII IX, X, XI and XII, von Willebrand Factor, Proteins C and S, thrombin, fibrinogen and mutants thereof.

The term "thrombolytics" has the generally accepted meaning in the art. As provided in the methods of the present invention, it refers to a polypeptide having thrombolytic activity, for example but not limited to staphylokinase (SAK), tissue-type plasminogen activator (t-PA), streptokinase (SK), urokinase (UK), urokinase-like plasminogen activator (u-PA), venom and mutants thereof.

The term "bone morphogenic protein" or "BMP" refers to any one of the family of growth factors or fragments thereof with the ability to promote bone growth, cartilage growth, or osteoblast differentiation and typically includes BMP-2, BMP-5, or BMP-7.

The term "engineered protein scaffold" has its generally accepted meaning in the art. As used in connection with the methods of the present invention, the engineered protein scaffold refers to a type of polypeptides having a structure with distinct biochemical functions and may comprise a plurality of structural domains and a plurality of loop regions that include an amino acid sequence that varies from a naturally-occurring loop region by at least one amino acid deletion, substitution, or addition. Generally, the structural domain or domains can include at least one $\beta$ structure or at least one $\alpha$ helix. For example, a review by Skerra, "Engineered protein scaffolds for molecular recognition," J. Mol. Recognit. 2000; 13:167-187 describes the following scaffolds: single domains of antibodies of the immunoglobulin superfamily, protease inhibitors, helix-bundle proteins, disulfide-knotted peptides and lipocalins. Further examples of engineered protein scaffolds in accordance with the present invention include inactivated staphylococcal nuclease, green fluorescent protein (GFP) and thioredoxin A (TrxA), the fibronectin type III domain ('Fn3'), lipocalin family proteins, bilin binding protein (BBP), as well as isolated protein folds such as the Z domain of staphylococcal protein A, "affibodies", anticalins, and ankyrin repeats, and others.

In connection with the present invention, the polypeptide may also be a fusion protein. The term "fusion protein" has its generally accepted meaning in the art. In accordance with the present invention, it particularly refers to a protein having at least two linked heterologous polypeptide portions. The term "heterologous" used in the context of the present invention indicates that said polypeptide portions are not found in the same relationship to each other in nature. The fusion protein is typically recombinantly produced, i.e. coding regions from different sources are ligated and the ligation product is expressed as the fusion protein.

The fusion protein used in the present invention may also be a protein comprising a tag. The term "tagged" or "protein tag" refers to a peptide sequence which is grafted onto a polypeptide and includes affinity tags, solubilization tags, chromatography tags and fluorescence tags. The polypeptide used in the present invention may be tagged, for example, for detection purposes. As used in the present invention, the polypeptide of interest can be tagged with a molecule to which an interaction member specifically binds. In a preferred embodiment of the invention, tags refer to epitope tags consisting of short sequences. It is known to the skilled person in the art that epitope tags are recognized by well-characterized antibodies. For example, suitable tags for antibodies and fragments thereof as used in accordance with the present invention include but are not limited to Myc tag, Flag-peptide tag, His Tag, Strep-Tag, GST-Tag, MBP- Tag, SNAP-Tag, Halo-Tag, Tap-Tag, INPACT-CN, pk or V5 tag, HA-tag and NE-tag. The cognate interaction members for each of these tags are known and can be used, for example, for detection and isolation of tagged proteins of interest. In a preferred embodiment of the invention, the tag is pk tag. The term "pk" and "V5" are used interchangeably herein and refers to a tag that is composed of a 14 residue peptide, GKPIPNPLLGLDST, derived from a small epitope (pk) present on the P and V proteins of the paramyxovirus of simian virus 5 (SV5). Preferably, the tag used in accordance with the present invention is a pK tag.

Preferably, fusion proteins used in the present invention are Fc fusion proteins. The term "Fc fusion protein" has its conventional meaning in the art and, as used in accordance with the present invention, particularly refers to a Fc region of an immunoglobulin or a functional portion thereof, which is referred to as the "Fc", that is fused to a moiety derived from a second, non-immunoglobulin protein. By the term "Fc" it is meant to include any part of the Fc-fusion protein in accordance with the present invention, which is derived from the Fc region of the immunoglobulin to form the Fc fusion protein. The fusion protein can be produced by a method of fusing a polynucleotide encoding an antibody of the present invention comprising the Fc portion and a polynucleotide encoding another peptide or polypeptide in-frame and inserting this into an expression vector, then expressing this in a host, and methods known to those skilled in the art may be used.

The terms "cell-free in vitro translation", "in vitro translation" (IVT), "cell-free translation", "in vitro protein expression" and "in vitro protein synthesis" are used interchangeably herein, and are intended to refer to any suitable method for the cell-free synthesis of RNA from a protein-coding DNA (transcription) and of a protein from the RNA (translation). In accordance with the present invention, IVT systems can be used which are known in the art and include, but are not limited to, those described in U.S. Pat. Nos. 5,665,563; 5,492,817; and 5,324,637, to Beckler et al., entitled "Coupled transcription and translation in eukaryotic cell-free extract"; U.S. Pat. No. 6,518,058 to Biryukov et al., "Method of preparing polypeptides in cell-free system and device for its realization"; U.S. Pat. No. 6,783,957 to Biryukov et al., entitled "Method for synthesis of polypeptides in cell-free systems"; United States Patent Application 2002/0168706 to Chatteijee et al., published Nov. 14, 2002, entitled "Improved in vitro synthesis system"; U.S. Pat. No. 6,548,276 to Swartz et al., issued Apr. 15, 2003, entitled "Enhanced in vitro synthesis of active proteins containing disulfide bonds"; United States Patent Application 2004/0110135 to Nemetz et al., published Jun. 10, 2004, entitled "Method for producing linear DNA fragments for the in vitro expression of proteins"; United States Patent Application 2004/0209321 to Swartz et al., published Oct. 21, 2004, entitled "Methods of in vitro protein synthesis"; United States Patent Application 2004/0214292 to Motoda et al., published Oct. 28, 2004, entitled "Method of producing template DNA and method of producing protein in cell-free protein synthesis system using the same"; United States Patent Application 2005/0032086 to Sakanyan et al., published Feb. 10, 2005, entitled "Methods of RNA and protein synthesis".

One prerequisite for in vitro protein synthesis is the production of a template comprising RNA or DNA. Templates for IVT used in accordance with the present invention refer to nucleic acid comprising RNA or DNA that encode the protein to be expressed and can be circular (inside plasmids, for example) or linear. In preferred embodiments of the methods of the present invention, RNA is messenger RNA (mRNA), which encodes proteins. In a particularly preferred embodiment, the template for IVT is DNA. As used in the present invention, the DNA template is preferably cDNA. The term "cDNA" means DNA that corresponds to or is complementary to at least a portion of messenger RNA (mRNA) sequence and is generally synthesized from an mRNA preparation using reverse transcriptase or other methods. Moreover, the term is often used to refer to cDNA sequences incorporated into a plasmid or viral vector which can, in turn, be present in a bacterial cell, mammalian packaging cell line, or host cell. Methods for producing cDNA templates are well known in the art and include the isolation of mRNA from a cellular source. General protocols are, for example, disclosed in Current Protocols in Molecular Biology, John Wiley & Sons, Ausubel et. al. eds., 1988, updated October 2001, Chapter 5, Construction of Recombinant DNA Libraries, particularly Section III, Preparation of Insert DNA from Messenger RNA. Methods for isolating RNA from eukaryotic and prokaryotic cells are well known in the art. For example, see Current Protocols in Molecular Biology, John Wiley & Sons, Ausubel et. al. eds., 1988, updated October 2001, Chapter 4, Preparation of RNA from Eukaryotic and Prokaryotic Cells. The initial mRNA may be present in a variety of different samples, where the sample will typically be derived from a physiological source. The physiological source may be derived from a variety of eukaryotic and prokaryotic sources. Physiological sources of interest include sources derived from single celled organisms such as yeast and multicellular organisms, including plants and animals, particularly mammals, preferably humans, primates and rodents, where the physiological sources from multicellular organisms may be derived from particular organs or tissues of the multicellular organism, or from isolated cells derived therefrom. In obtaining the sample of RNAs from the physiological source from which it is derived, the physiological source may be subjected to a number of different processing steps, where such processing steps might include tissue homogenization, cell isolation and cytoplasmic extraction, nucleic acid extraction and the like, where such processing steps are known to those of skill in the art. Eukaryotic and prokaryotic sources include, but are not limited to, bacteria, plant, fungi, insect and mammalian sources. Preferred sources of RNA for use in the present invention are human, rodent, and primate.

Once isolated, mRNAs are then used as template for the synthesis of double stranded cDNA (dscDNA) using the enzyme reverse transcriptase. Synthesis of cDNA may be done in vitro or in vivo, as known from methods known in the art (for example, see U.S. Pat. No. 5,891,637, issued 6 Apr. 1999 to Ruppert et. Al). In the process of converting mRNA into double stranded cDNA in vitro, a first cDNA strand is synthesized by the reverse transcriptase. A DNA polymerase, such as E. coli DNA polymerase, then uses the first cDNA strand as a template for the synthesis of the second cDNA strand, thereby producing a population of double stranded DNA molecules. First strand cDNA synthesis is performed using any convenient protocol. In preparing the first strand cDNA, a primer is contacted with the mRNA, a reverse transcriptase, and other reagents necessary for primer extension under conditions sufficient for first strand cDNA synthesis to occur. The use of primers in cDNA synthesis is well known in the art. Additional reagents that may be present include: dNTPs; buffering agents, e.g. TrisCl; cationic sources, both monovalent and divalent, e.g. KCl, $MgCl_2$; sulfuhydryl reagents, e.g. dithiothreitol; and the like. A variety of enzymes, usually DNA polymerases, possessing reverse transcriptase activity can be used for the first strand cDNA synthesis step. The double stranded cDNA is ligated to adaptors, and adaptor-modified cDNA is subsequently directionally cloned into expression vectors. "Expression vector" includes vectors of the present invention which are capable of expressing cDNA sequences contained therein. An expression vector typically contains a promoter in reading frame with the gene and compatible with the proposed cell extract. A number of vectors such as those are described in U.S. Pat. Appln. Ser. Nos. 307473; 291892; and 305657 (EPO Publ. Nos. 0036776; 0048970 and 0051873).

Examples of expression vectors derived from *E. coli* include pGEX-5X-1 (manufactured by Pharmacia), "QIAexpress system" (manufactured by QIAGEN), pEGFP, and pET (in this case, the host is preferably BL21 expressing T7 RNA polymerase). In addition to *E. coli,* a vector for producing a protein or polypeptide of the present invention may be, for example, expression vectors derived from mammals (e.g., pcDNA3 (manufactured by Invitrogen), pEF-BOS (Nucleic Acids Res. 1990, 18(17), p5322), pEF, pCDM8), expression vectors derived from insect cells (e.g., "Bac-to-BAC baculovirus expression system" (manufactured by GIBCO-BRL), pBacPAK8), expression vectors derived from plants (e.g., pMH1, pMH2), expression vectors derived from animal viruses (e.g., pHSV, pMV, pAdexLcw), expression vectors derived from retroviruses (e.g., pZIPneo), expression vectors derived from yeasts (e.g., "Pichia Expression Kit" (manufactured by Invitrogen, pNV11, SP-Q01), and expression vectors derived from *Bacillus subtilis* (e.g., pPL608, pKTH50).

In a particularly preferred embodiment of the invention, the cDNA template is cloned into the pT7CFE1-Nhis-GST-CHA vector or a pT7CFE1 vector derivative thereof. It is to be understood that cDNA encoding mutated polypeptides to be screened according to the present invention generated by site-directed mutagenesis, can be correspondingly obtained by methods as described above. Hence, a cDNA library containing the wild-type and variants of the mutated polypeptide of the present invention can be prepared from mRNA using suitable primers and at least one point mutation of the generated cDNA is introduced, preferably by site-directed mutagenesis as described above.

Methods to amplify DNA templates are well known in the art and are commonly based on the PCR method. As it belongs to prior art knowledge, PCR allows for replicating/amplifying trace amounts DNA fragments from a template. Briefly, a typical PCR reaction includes three steps: a denaturing step in which a target nucleic acid is denatured; an annealing step in which a set of PCR primers (forward and backward primers) anneal to complementary DNA strands; and an elongation step in which a thermostable DNA polymerase elongates the primers. By repeating this step multiple times, a DNA fragment is amplified to produce an amplicon, corresponding to the target DNA sequence. Typical PCR reactions include 30 or more cycles of denaturation, annealing and elongation. In many cases, the annealing and elongation steps can be performed concurrently, in which case the cycle contains only two steps. The specificity of any given PCR reaction relies heavily, but not exclusively, on the identity of the primer sets. The primer sets are pairs of forward and reverse oligonucleotide primers that anneal to a target DNA sequence to permit amplification of the target sequence, thereby producing a target sequence-specific amplicon.

In IVT systems used for the present invention wherein the input template nucleic acid molecule is linear DNA, the components necessary both for transcription (to produce mRNA) and for translation (to synthesize protein) can be contained in a single IVT system. Common components of a cell-free translation system necessary both for transcription (to produce mRNA) and for translation (to synthesize protein) of DNA in IVT systems are known in the art. In accordance with the present invention, such components present in cell extracts include, for example, but are not limited to a cell extract from HeLa cells, CHO cells, yeast, rabbit reticulocytes, wheat germ and E. coli. The extracts are prepared as crude extracts containing all the required macromolecular components for translation of exogeneous RNA including 70S or 80S ribosomes, tRNAs, aminoacyl-tRNA synthetases, translation initiation and elongation factors, nucleases, etc.. To ensure efficient translation, each extract is generally supplemented with energy sources (ATP, GTP), amino acids, co-factors (magnesium, potassium, etc.) and the DNA template cloned into a vector. As known to the skilled person, a vector containing a T7 promoter and an EMCV Internal Ribosome Entry Site (IRES) can facilitate high levels of *in vitro* protein expression. Hence, in a preferred embodiment of the invention, the vector of the present invention contains a T7 promoter and an EMCV Internal Ribosome Entry Site (IRES).

In alternate methods of the present invention, additional enzymes, substrates and/or co-factors for post-translational modification can optionally be added to a cell extract comprising the template to be transcribed and translated in an IVT system.

If necessary, a purification step can be performed prior to IVT of the DNA template. IVT can be preceded by a purification step in order to free the nucleic acid from unwanted cellular contaminants which may interfere with subsequent processing.

Various methods to purify vector DNA include but are not limited to isolating and purifying plasmid DNA include lysis by boiling (Holmes and Quigley, Anal. Biochem. 114, 193 (1981)), lysis with alkali (Birnboim and Doly, Nuc. Acids Res. 7, 1513 (1979)), and lysis with detergent (Godson and Vapnek, Biochem. Biophys. Acta 299, 516 (1973)). Commonly used mini-scale plasmid DNA extraction protocols exploit reagents originally developed by Birnboim and Doly (Birnboim, H. C. and Doly, J., Nucl. Acids Res. 7, 1513 (1979). For mini-scale purification, the clarified solution is usually applied to a microspin column containing a glass fiber matrix or silica membrane. Vector DNA binds to the column in the presence of a chaotrope, while soluble impurities do not bind. After the soluble impurities are washed off, the plasmid DNA are eluted with an appropriate elution buffer. In a preferred embodiment of the invention, a mini-prep protocol is used for the purification of the vector DNA.

In accordance with the present invention, the preparation of cell extracts that support the synthesis of proteins *in vitro* from purified mRNA transcripts, or from DNA transcribed into mRNA during the *in vitro* synthesis reactions are well known in the art and include both prokaryotic and eukaryotic systems, which are commercially available.

Prokaryotic extracts to be used in the present invention encompass extracts from any prokaryotic cells, including, without limitation, gram negative and gram positive bacteria, including *Escherichia* sp. (e.g., *E. coli*), *Klebsiella* sp., *Streptomyces* sp., *Streptococcus* sp., *Shigella* sp., *Staphylococcus* sp., *Erwinia* sp., *Klebsiella* sp., *Bacillus* sp. (e.g., *B. cereus, B. subtilis* and *B. megaterium*), *Serratia* sp., *Pseudomonas* sp. (e.g., *P. aeruginosa* and *P. syringae*), *Salmonella* sp. (e.g., *S. typhi* and *S. typhimurium*), and *Rhodobacter* sp. Standard IVT systems may be made from *E. coli* lysates. Cell extracts can be made from bacterial strains mutated to lack a nuclease or protease activity, or to lack the activity of one or more proteins that can interfere with purification or detection of translated proteins (see U.S. Patent Publication No. US2005/0136449, herein incorporated by reference in its entirety).

Eukaryotic extracts to be used in the present invention for include without limitation rabbit reticulocyte lysates, wheat germ lysates, *Drosophila* embryo extracts, insect cell lysates, rabbit oocyte lysates and human cell lysates (Zubay (1973), Pratt (1984), and U.S. Pat. No. 5,665,563, Pelham et al, European Journal of Biochemistry, 67: 247, (1976) among others). Some of these extracts and lysates are available commercially (Promega, Madison, Wis.; Agilent, La Jolla, Calif.; GE Healthcare, Arlington Heights, III.; Thermo Fisher Scientific, Grand Island, N.Y.). Various embodiments of the current invention will be applicable to any IVT systems, including, but not limited to HeLa cell lysates, CHO cell lysates, reticulocyte lysates and wheat germ extracts. In a particularly preferred embodiment of the invention, the cell extract is HeLa cell lysate.

In particular, the DNA template used for the present invention is transcribed and translated simultaneously in a coupled IVT system.

Coupled IVT systems are also commercially available and can be used in accordance with the methods of the present invention. One example is the RTS™ system (Rapid Translation System) of Roche Biochemicals (Germany) which uses *E. coli* extracts and employs continuous exchange cell-free system (CECF) and an improved energy-regeneration system (Kim et al., Biotechnol Bioeng., 74(4):309-16 (2001)). Other examples of commercially available IVT systems that can also be used in the invention include IVT Kits of Thermo Scientific (Rockford, Illinois) ProteinScript PRO™ of Ambion (Austin, Tex.), and TNT® system of Promega (Madison, Wis.), among others.

Detection and quantification of expressed proteins used for the present invention can be performed by methods well known in the art and include but are not limited to SDS-polyacrylamide gel electrophoresis, capillary electrophoresis, limited proteolysis and tandem array mass spectrometry, enzyme assay, cell-based assays and a wide of immunological techniques such as Western blotting and ELISA. In a preferred embodiment of the invention, such methods would be simple, inexpensive and rapid, with high sensitivity and specificity for the target molecule to be detected.

In a preferred embodiment of the methods of the present invention, the polypeptide expressed by cell-free *in vitro* translation comprises less than 1 μg of active polypeptide, preferably less than 100 ng of active polypeptide, and more preferably less than 10 ng of active polypeptide. This preferred embodiment reflects the surprising beneficial finding of the invention that, by the use of solution titration followed by sandwich immuno-assay, such as ELISA, for activity determination, the screening can be done in an efficient and highly specific manner using low amounts of and less purified polypeptides. The term "active polypeptide" as used in accordance with the present invention refers to the polypeptide fraction in a preparation that is able to perform a biological function, i.e. that the polypeptide has a conformation ("folded form") which is the same as or closely analogous to its naturally occurring configuration. When folded correctly or substantially correctly, for example, with the formation of properly folded units, α-helices, β-sheets, domains, disulphide bridges, etc., a polypeptide generally has the ability to perform its biological function. Methods for detection and quantification of active polypeptides expressed by IVT are well known in the art and include, e.g., methods which depend on having an antibody ligand for the protein, such as Western blotting (see, e.g., Burnette, 1981, A. Anal. Biochem. 112:195-203). Such methods also include enzymatic activity assays, which are available for most well-studied protein drug targets, including, but not limited to, HMG CoA reductase (Thorsness et al., 1989, Mol. Cell. Biol. 9:5702-5712), and calcineurin (Cyert et al., 1992, Mol. Cell. Biol. 12:3460-3469). In a particularly preferred embodiment of the invention, expressed active proteins are detected and quantified by Western blotting.

As used in the present invention, the concentration of the active polypeptide expressed by cell-free *in vitro* translation in a cell lysate comprises up to 10 % (w/v) and more preferably, the concentration of the active polypeptide expressed by cell-free *in vitro* translation in a cell lysate comprises up to 7 % (w/v) and even more preferably, the concentration of the active polypeptide expressed by cell-free *in vitro* translation in a cell lysate comprises up to 5 % (w/v). In a preferred embodiment of the methods of the present invention, the active polypeptide expressed by cell-free *in vitro* translation comprises up to 5% (w/v) of the lysate. The terms "lysate", "cell lysate" and "cell extract" are used interchangeably herein and refer to preparations obtained by lysing cells as well as functional fractions thereof. Cell lysates typically comprise a complex mixture of constituents such as proteins, glycoproteins, polysaccharides, lipids, nucleic acids etc. A cell lysate for use in the present invention may comprise the entire cell material, parts of cells or any fractions or mixtures thereof obtained after a lysis step. The concentration of the active polypeptide expressed by cell-free *in vitro* translation in a cell lysate used for the present invention varies according to the type of cell lysate. As used in the present invention, the percentage of the active polypeptide expressed by cell-free *in vitro* translation in a cell lysate refers to the weight percentage of total protein in the cell lysate, as determined by a suitable protein quantification assays as described above well known in the art (see, e.g., Schena et al., 1995, Quantitative monitoring of gene expression patterns with a complementary DNA micro-array, Science 270:467-470; Lockhort et al., 1996, Expression monitoring by hybridization to high-density oligonucleotide arrays, Nature Biotechnology 14:1675-1680; Blanchard et al., 1996, Sequence to array: Probing the genome's secrets, Nature Biotechnology 14, 1649; 1996, U.S. Pat. No. 5,569,588, issued Oct. 29, 1996 to Ashby et al. entitled "Methods for Drug Screening").

IVT systems used in accordance with the present invention can preferably be applied in a high-throughput setting, where preferably at least two of the templates are processed simultaneously in multi-well reaction plates, where each nucleic acid template is in a well of the plate. The reaction plate has preferably at least 2 wells, and even more preferably 12-, 24-, 48-, 96-, 384-, or 1536-wells; other sizes may also be used.

Optionally, the polypeptide expressed by IVT can be purified. Methods for purification of proteins are commonly known in the art and are not limited to any method. Polypetides expressed by IVT may be purified by appropriately selecting and combining, for example, column chromatography, filtration, ultrafiltration, salting out, solvent precipitation, solvent extraction, distillation, immunoprecipitation, SDS-polyacrylamide gel electrophoresis, isoelectric focusing, dialysis, and recrystallization. Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., Cold Spring Harbor Laboratory Press, 1996). Such chromatography can be carried out using liquid phase chromatography such as HPLC and FPLC. Columns used for affinity chromatography include protein A column and protein G column, Nickel_NTA columns for purifying His-tagged proteins and GST columns for purifying GST-tagged proteins. Examples of columns using protein A column include Hyper D, POROS, and Sepharose FF (GE Healthcare Biosciences). Preferably, the methods of the present invention are carried out without purifying the polypeptide expressed by IVT.

The term "screening the activity of a polypeptide" as used in accordance with the present invention refers to a procedure in which a property of one or more polypeptides is determined by expressing a polypeptide and determining the interaction activity between a polypeptide, for example an antibody, and an interaction member, for example an antigen using IVT, wherein the activity to be screened is the binding affinity of the polypeptide to the interaction member. The term "binding" according to the invention preferably relates to specific binding. "Specific binding" means that a polypeptide of the invention binds stronger to an interaction member for which it is specific compared to the binding to another non-polypeptide target.

It is to be understood that the screening of the binding activity of a polypeptide to an interaction member includes both the expression of a polypeptide to be screened and the measurement of the activity of said polypeptide. In the context of the present invention, the expression of a polypeptide to be screened is performed by IVT as described above. In a preferred embodiment of the invention, the expressed polypeptide to be screened is any of the polypeptides described above. In a particularly preferred embodiment, the screening comprises the screening of a library of polypeptide variants.

In the context of the present invention, solution equilibrium titration is used as the assay for "determining the activity" of the expressed polypeptide with an interaction member, wherein said activity is the binding activity of the polypeptide to the interaction member.

Solution equilibrium titration methods are known in the art and are described in further detail below. As provided in the present invention, a solution equilibrium titration assay refers to a method for determining the binding affinity of a polypeptide to an interaction member. Solution equilibrium titration can be performed as demonstrated in the Examples set out below. Solution equilibrium titration for screening the interaction of antibodies or fragments thereof with their relevant binding partners is for example described in Heanel and Coworkers in 2005 and has been applied in different groups since then (Gustchina et al., Virology, 393:112-119 (2009); Huber et al., J. Struct. Biol. 159:206-221 (2007)). The starting point of this method is to measure a protein's binding affinity to its interaction member by titration with an interaction member. Thereby, a constant concentration of polypeptide is incubated with decreasing concentrations of the interaction member in solution. As the polypeptide will bind to the interaction member and some polypeptides will remain free in solution, equilibrium of the polypeptide - interaction member reactions is reached. The portion of free polypeptide can be quantified in a second assay step, wherein the equilibrated samples are briefly incubated on a plate coated with the interaction member, allowing binding of the free polypeptide to the coated interaction member. After washing the coated plate to remove unbound material, the concentration of the polypeptide can be determined by well-known detection methods as described in more detail below. Subsequently, an equilibrium dissociation constant, $K_D$, can be determined taking the applied concentrations of polypeptide and interaction member into consideration. The $K_D$ is a well known constant used as a measure for binding affinity between a polypeptide and an interaction member. Thus, the binding affinity of said interaction polypeptide in relation to the binding member and more preferably the binding affinities of multiple polypeptides or polypeptide variants that all bind the same interaction member can be determined by determining their corresponding $K_D$. These measurements can then be used to rank the binding affinities, as for example shown by Della Ducata et al. (Journal of Biomolecular Screening, 20(10): 1256-1267, 2015)) describing a SET setting for high-throughput affinity estimation of unpurified antibodies and fragments. In this method the concentration of expressed active constructs was measured based on a standard immunoassay standard curve approach. Based on this della Ducata et al show that using SET with only 4 titration points for the ligand of the ligand-antibody pair investigated in that publication, ranging through an interval of 5-10-fold below the $K_D$ of the wild type to at least 10-20 times above said $K_D$, a reasonably accurate $K_D$ can be determined for most mutants in high throughput. These methods are therefore equally applicable to the present invention.

As readout system for the SET assay preferably chemiluminescence or fluorescence, or electrochemiluminescence (ECL) is used, e.g., as described by Haenel and coworkers in 2005 (Anal. Biochem. 339: 182-184). Preferably, the SET assay, as used in accordance with the present invention, is transferred from a bead-based approach to a plate-based method such as the plate-based Meso Scale Discovery (MSD; Meso Scale Diagnostics, Rockville, MD) platform. The principle of the general plate-based or MSD-based SET assay is based on incubation of a constant concentration of protein or peptide with decreasing concentrations of the interaction member in solution which is carried out in a multi-well assay plate. When the interaction between the polypeptide and the interaction member is equilibrated, the portion of free antibody is transferred to the relevant binding plate that can be coated with an antigen, to allow for binding of the free polypeptide to the coated antigen. For detection, a labeled secondary antibody is added to the plate which is suitable for detection e.g. by fluorescence, chemiluminescence or electrochemiluminisecence. Most preferably, chemiluminescence is used in accordance with the present invention. Thereby, the intensity of the emitted signal can be quantified as a directly proportional measure of concentration of free polypeptide. From the resulting titration data, the $K_D$ value can be determined taking into account the concentration of free polypeptide for each sample. The SET screening platform is particularly applicable for screening approaches for reliably determining the $K_D$ of complex samples, such as antibody fragments and full length antibodies in unpurified matrices, and ranking of the binding affinity. Furthermore, the typical throughput of an affinity SET screening campaign is approximately 100-2000 antibodies within two days including data evaluation. This is rather rapid compared to other known screening platforms (SPR or BLI) (Lad et al., J. Biomol. Screen., 20:498-507 (2015); Ylera et al., Anal. Biochem., 441:208-213 (2013); Canziani et al., Anal. Biochem., 325:301-307 (2004)). On the one hand SET can be carried out easily using dozens of multi-well microplates for which efficient dispensing, handling and plate reading equipment exists. Compared to SPR and BLI the SET method, which essentially relies on a sandwich immuno-assay principle, is much more tolerant to analyzing crude samples.

The term "determining the activity of the polypeptide" used in accordance with the present invention refers to determining the binding affinity of a polypeptide to be screened to an interaction member by solution equilibrium titration. As used in accordance with said preferred embodiment, the binding affinity is characterized by the strength of the binding interaction between a polypeptide and its interaction member, wherein the binding affinity is generally quantified by the solution equilibrium dissociation constant, $K_D$. The term "equilibrium dissociation constant" refers to a constant that describes the binding affinity between binding partner molecules at equilibrium relating the proportion of the products of the concentrations of unbound binding partner molecules to the concentration of the bound binding partner complexes. It describes the state of equilibrium between free and bound polypeptide. The term "state of equilibrium" refers to the state wherein the concentrations of the free and bound polypeptide each remain constant. The term "bound polypeptide" as used in accordance with the present invention refers to a polypeptide which is bound to an interaction member. The equilibrium dissociation constant $K_D$ is a term well known in the art and described in detail in the following Wikipedia article https://en.wikipedia.org/wiki/Dissociation constant where it is referred to as $K_d$.

In a preferred embodiment of the invention, a $K_D$ that is not substantially altered varies less than 10-fold more preferably less than 3-fold Alternatively, the $K_D$ of the polypeptide with a desired activity to an interaction member refers to an affinity that is altered.

In a preferred embodiment of the invention, the binding affinity of the polypeptide is measured by SET and subsequently the $K_D$ is determined by a detection suitable detection method known to the skilled person in the art, for example but not limited by fluorescence, chemiluminescence, or electrochemiluminescence (ECL).

In a preferred embodiment of the methods of the invention, the $K_D$ of the binding affinity of the polypeptide is less than 1 nM, preferably less than 100 pM and more preferably less than 10 pM. In a further preferred embodiment of the invention, the $K_D$ is less than 1 pM

As used in accordance with the present invention, the term "interaction member" refers to a molecule that interacts with another molecule. According to the present invention, such other molecule specifically or non-specifically binds to the interaction member. In a preferred embodiment of the invention, such other molecule specifically binds to the interaction member. Binding, in particular specific binding, is mediated by physical forces, for instance electrostatic attraction, hydrogen bonds or van der Waals bonds, resulting in binding of the interaction member to the other ligand. A particularly preferred interaction member, as used in the methods according to the present invention, could be a protein in the cytosol of a cell. Another particularly preferred interaction member could be a membrane protein. However, the interaction member not necessarily needs to be a protein, but can be a nucleic acid, a lipid, a carbohydrate, a combination of any of the afore-mentioned compound classes, or any other kind of molecule. For example, an interaction member includes antibodies (forming an antibody/epitope complex), antigens, nucleic acids (e.g. natural or synthetic DNA, RNA, cDNA, mRNA, tRNA, etc.), lectins, sugars (e.g. forming a lectin/sugar complex), glycoproteins, receptors and their cognate ligand (e.g. growth factors and their associated receptors, cytokines and their associated receptors, signaling receptors, etc.), small molecules such as drug candidates (either from natural products or synthetic analogues developed and stored in combinatorial libraries), metabolites, drugs of abuse and their metabolic by-products, co-factors such as vitamins and other naturally occurring and synthetic compounds, oxygen and other gases found in physiologic fluids, cells, cellular constituents, cell membranes and associated structures, inter-cellular spaces, including serum and plasma, other natural products found in plant and animal sources, other partially or completely synthetic products, and the like. In a preferred embodiment of the invention, the interaction member is VEGF.

The term "producing a polypeptide with a desired activity" refers to the method of the present invention capable of identifying a polypeptide variant with a desired activity by producing a set of protein variants and selecting for the desired activity by applying the screening method of the invention comprising step (i) and (ii), wherein the desired activity is a desired binding affinity. Thus, the production method may comprise a step of devising a set of protein variants, said variants preferably differing from each other by point mutations, which precedes said steps (i) and (ii).

In a preferred embodiment of the methods of the present invention, the methods comprise the expression and activity determination of at least 100 polypeptide variants. The variant may have "conservative" changes, wherein a substituted amino acid has similar structural or chemical properties (e.g., replacement of leucine with isoleucine). A variant may also have "nonconservative" changes (e.g., replacement of glycine with tryptophan). Preferably, the variation at one particular position of the amino acid sequence comprises all 20 standard amino acids. Analogous variations may also include amino acid deletions or insertions, or both. Preferably a variant of a polypeptide has at least 60% identity to the wild-type polypeptide. More preferably a variant of a polypeptide is at least 70% identical to the wild-type polypeptide. Protein variants can be, for example, at least 80%, at least 90%, at least 95%, or at least 99% identical to wild-type polypeptide. Preferably, the polypeptides of a mutant library comprise at least 300 polypeptide variants, more preferably at least 500, and even more preferably at least 1000 polypeptides.

In a preferred embodiment of the methods of the present invention, the expressed polypeptide or its interaction member contains a binding domain of an antibody fragment. The term "binding domain" refers to the part of an antibody fragment which comprises the area which specifically binds to part or all of the expressed polypeptide or its interaction member. Where the expressed polypeptide or its interaction member is large, an antibody fragment may bind to a particular part of the interaction member only, which part is termed an epitope.

In a preferred embodiment of the methods of the present invention, the expressed polypeptide or its interaction member contains the active domain of an enzyme, or the active domain of a substrate of an enzyme, or the active domain of a regulatory protein of an enzyme. The term "the active domain" as used in accordance with the present invention refers to the portion of the protein, which contains a region that is required in the protein in order to participate in the function that is the object of interest to the investigator. In a particularly preferred embodiment of the invention, the active domain is a domain that participates in a corresponding enzyme reaction.

In the context of the present invention, said expressed polypeptide or interaction member of said enzyme may be a substrate or regulatory protein of the enzyme with one or more introduced point mutations, wherein the affinity and/or turn-over rate of said substrate or regulatory protein with one or more introduced point mutations to the enzyme may be increased compared to the affinity and/or turn-over rate by the reaction with the wild-type substrate. Thereby, the mutated substrate and/or regulatory protein may impair the binding and catalysis of the wild-type substrate due to the higher binding affinity. In addition, since the turn-over rate by an enzyme may be a measure for enzyme activity, such enzyme activity may be increased by said substrate or regulatory protein with one or more introduced point mutations.

In a preferred embodiment of the methods of the present invention, the screening comprises identifying a polypeptide with an affinity to an interaction member that is altered as a consequence of one or more introduced point mutations. The term "altered affinity" as used in the methods of the present invention refers to a polypeptide with a higher or lower affinity to in interaction member due to one or more introduced point mutations. Methods to generate polypeptides with one or more introduced point mutations are known in the art and described above. Methods to measure the affinity of a polypeptide are well known in the art and described above. Furthermore, the term "identifying a polypeptide with an affinity to an interaction member" refers to the identification of the binding affinity of a polypeptide determined by the $K_D$ of the polypeptide to an interaction member as provided by the methods of the present invention or known in the art and described above. When a mutant polypeptide has a lower binding affinity to its interaction member compared to a reference polypeptide, the methods of the present invention are useful to generate mutant polypeptide variants having a higher binding affinity than the reference polypeptide. The reference polypeptide preferably is the wild-type polypeptide from which the mutant polypeptide has been derived. As an alternative embodiment, the screened mutant polypeptide may also be derived from another mutant polypeptide. Preferably, the $K_D$ of the binding affinity of a polypeptide to an interaction member is increased or decreased at least 10-fold, preferably at least 20-fold, more preferably at least 50-fold, even more preferably at least 100-fold, 200-fold, 500-fold, or 1000-fold than the $K_D$ of the binding affinity of the reference polypeptide to the interaction member. It is particularly desirable to identify polypeptides with an increased affinity to their interaction member as a consequence by one or more point mutations, in particular in connection with the development of a therapeutic application.

In a preferred embodiment of the methods of the present invention, the screening comprises identifying a polypeptide with an affinity to an interaction member that is not substantially altered as a consequence of one or more introduced point mutations.

As provided by the methods of the present invention, the identification of the binding affinity of a polypeptide is determined by the $K_D$ of the polypeptide to an interaction member. The term "not substantially altered" of the present invention denotes a change of the $K_D$ that does not affect the desired interaction of a polypeptide with an interaction member in a noticeable way to the practitioner. In a preferred embodiment of the invention, the binding affinity refers to $K_D$ that is not substantially altered varies less than 10-fold more preferably less than 3-fold. This preferred embodiment is particularly useful in situations where the binding affinity of the polypeptide is already at a desirable level and where one or more point mutations are being introduced for reasons other than modifying the affinity, such as for removing a T cell epitope potentially inducing an immune response in a patient.

Following the same goal, in a preferred embodiment of the methods of the present invention, the screening comprises identifying a polypeptide with an affinity to an interaction member that is either increased or not substantially altered as a consequence of one or more introduced point mutations and wherein one or more of said point mutations reduces the binding of a sub-sequence of the polypeptide comprising at least one of said mutations to an MHC molecule. The terms "MHC molecule" and "Major Histocompatibility Complex molecule" are used interchangeably in the present invention and refer to molecules which are found on the cell surface in tissues, play an important role in presenting cellular antigens in the form of short linear peptides to T cells by interacting with T cell receptors (TCRs) present on the surface of T cells. MHC molecules typically contain three "constituent peptides": an MHC alpha and MHC beta chain, and an MHC binding peptide bound in a groove formed by the alpha and beta chains when properly folded. In the above interaction of the MHC molecule and the T cell receptor, the T cell recognizes and binds to the peptide bound in the MHC binding groove and the surrounding area of the MHC binding groove itself. In order for a peptide to bind to an MHC molecule and therefore be able to cause an immune response *in vivo*, it needs to fit the properties of the binding groove. Understanding, which peptides in a protein sequence can bind to a given MHC molecule and cause an immune response, is of considerable interest for understanding cellular immunity and in order to design and monitor the effectiveness of immunotherapeutic products. It is of particular interest of the present embodiment to reduce the binding affinity of a polypeptide to an MHC molecule as a low affinity may avoid unwanted immunogenicity. Preferably, the $K_D$ of the sub-sequence of the polypeptide with an MHC molecule when compared to a reference sub-sequence is reduced by at least 10-fold, preferably at least 20-fold, more preferably at least 50-fold, even more preferably at least 100-fold, 200-fold, 500-fold, or 1000-fold compared to the $K_D$ of the binding affinity of a reference sub-sequence to the MHC molecule. The reference sub-sequence of the polypeptide preferably is the wild-type sub-sequence of the polypeptide from which the mutant sub-sequence has been derived. As an alternative embodiment, the screened mutant sub-sequence may also be derived from another mutant polypeptide. Methods to determine the affinity of a sub-sequence of a polypeptide binding to an MHC molecule are well known in the art and described above.

In a preferred embodiment, the methods of the present invention further comprise screening for a polypeptide with a cross-reactivity between more than one interaction member as a consequence of one or more introduced point mutations in said polypeptide. In the context of the present invention, the term cross-reactivity refers to the capability of the polypeptide, preferably an antibody or fragment thereof, to recognize and specifically bind to more than one interaction member. Preferably, a cross-reactive polypeptide binds to more than one interaction member with a $K_D$ of 100 nM or less, 10 nM or less, 1 nM or less, 100 pM or less, or 10 pM or less. Examples for cross-reactivity between a polypeptide and more than one interaction member can be found in the following references: (Gotch et al, 1988, J Exp Med., 168(6):2045-57; Petrova et al., 2011, J Immunol., 186(11):6390-7; Kan-Mitchell et al., 2006, J Immunol. 176(11):6690-701; Vali et al, 2011, J Virol., 85(1):254-63; Acierno et al., 2003, J Transl Med., 14;1(1):3; Wedemeyer et al., 2001, J Virol., 75(23):11392-400; Kasprowicz et al., 2008, J Clin Invest., 118(3):1143-53). For example, T-cell cross-reactivity is a phenomenon of the immune system defined as the recognition of two or more peptide-MHC complexes (pMHCs) by the T cell receptor (TCR). The recognition is based on the ability of the TCR to bind sufficiently well to initiate a cellular response. The achievement of cross-reactivity may be useful in the context of drug development. For instance, in the case of a therapeutic antibody directed to a human target, it may be useful to achieve cross-reactivity to the homologous target in a pre-clinical model close to the human system, such as in non-human primates. In order for such a pre-clinical model to be properly valid, however it is highly desirable that the mode of action of the antibody or drug is replicated in the primate model. For drugs and antibodies that have inadequate cross-reactivity there may therefore not be an adequate path to establish non-clinical safety. If however the antibody can be re-engineered to increase cross-reactivity to an equivalent non-human primate target then the sufficiency of the non-clinical safety model can be improved and facilitate the clinical development of drug programs that would otherwise not be feasible.

In a preferred embodiment, the methods of the present invention further comprise the screened polypeptide(s) having an increased half-life *in vivo* as a consequence of one or more introduced point mutations in said polypeptide(s). The term "half-life" refers to the time taken by the polypeptide to be screened to be removed from the blood stream. Methods to determine the half-life of a polypeptide are well known in the art and include metabolic pulse-chase with isotopically labeled amino acids (Dickson and Mendenhall, 2004), measurement of isotopically labeled amino acids of a polypeptide by mass spectrometry (Bateman etal., 2006; Mawuenyega etal., 2010). Small size polypeptides are often rapidly cleared from the blood stream (Batra et al., 2002; Jain et al., 2007). Consequently, it is of particular interest for the application of therapeutic polypeptides to extend the plasma half-life of these molecules. As provided by technologies known in the art, an increase of the binding affinity of a polypeptide of interest leads to an increased half-life (Hinton et al., J Immunol., 176(1):346-56 (2005); Dall'Acqua et al., J. Biol. Chem. 281(33):23514-24 (2006); Johansson et al., J. Biol. Chem, 277:8114-8120 (2002); Linhult et al., Protein Sci. 11: 206-213 (2002); Zalevsky et al., Nat. Biotechnol. 28(2): 157-159 (2010)). In this regard, only the unbound fraction of a polypeptide used as a drug in a patient undergoes degradation resulting in a reduced half-life of that drug. As the drug dissociates from the intended interaction member the drug undergoes metabolism in the liver and other tissues. In turn, a higher affinity of the drug would lead to a higher fraction of drug bound to the interaction member thereby typically increasing the half-life of a drug.

In a preferred embodiment of the methods of the present invention, the increased half-life *in vivo* as a consequence of one or more introduced point mutations in said polypeptide(s) is due to an altered binding affinity of said polypeptide(s) to FcRn. The term "FcRn" as used in accordance with the present invention refers to the neonatal Fc receptor which is a membrane-associated receptor involved in both IgG and albumin homeostasis, in maternal IgG transport across the placenta and in antigen-IgG immune complex phagocytosis. FcRn structure consists of three extracellular alpha ($\alpha$) domains, a single pass transmembrane domain, and a short cytoplasmic tail of approximately 44 amino acids. It was shown that increased binding affinity for FcRn increases the half-life of an IgG, the most prevalent immunoglobulin class in human and other mammals (see for example, Kim et al., Eur J Immunol., 24:2429 (1994); Kuo et al (2010), Journal of Clinical Immunology. 30 (6): 777-789; Zalevsky et al., 2010 nature Biotechnology 28(2):157-159). For example, Kuo et al. (2010) describes the ability of FcRn to prolong the half-life of IgG and albumin, which has guided engineering of novel therapeutics. In this regard, two FcRn molecules bind to a single IgG with a 2:1 stoichiometry, and the binding occurs at pH 6.0-6.5 with minimal structural conformation change and not at pH 7.5. Site-directed mutagenesis studies have proven that the critical binding sites in the Fc domain of IgG for FcRn are Histidine 310, Histidine 435, and Isoleucine 253 and to a lesser extent Histidine 433 and Tyrosine 436. At acid pH, the protonated histidine residues on IgG form a salt bridge with the FcRn $\alpha$2 domain at Glutamic acid 117, Glutamic acid 132, and/or Glutamic acid 135 and/or Aspartic acid 137. Therefore, FcRn can bind IgG from the slightly acidic intestinal lumen and ensure efficient, unidirectional transport to the basolateral side where the pH is neutral to slightly basic. Hence, it is of particular interest of the present embodiment to increase the half-life of a polypeptide that is intended for the development of a therapeutic protein.

## Examples

The present invention will be further illustrated by way of the following examples, which are however not intended to limit the scope of the claims.

## Example 1

### 1.1 Expression of wild-type and mutant Avastin® (bevacisumab) by coupled cell-free *in vitro* transcription/ translation (IVT) system

DNA encoding wild-type (wt) or mutant Avastin (Av) scFvs were expressed using a cell-free *in vitro* coupled transcription/translation (IVT) system. A C-terminal affinity tag was added to the scFv construct to enable later detection by ELISA.

Table 1. List of UPH1-6 constructs and the mutations they contain from the wt Av scFv, wherein the numbering of the mutated positions all refer to the amino acid position in the wt Av heavy chain. The full heavy and light chain sequences for Avastin are readily available from Drugbank: https://www.drugbank.ca/drugs/DB00112

| Name | Construct |
|------|-----------|
| UPH1 | Avastin scFv |
| UPH2 | [T28D/N31H/H101Y/S105T] Avastin scFv |
| UPH3 | [T28D] Avastin scFv |
| UPH4 | [N31H] Avastin scFv |

(continued)

| Name | Construct |
|------|-----------|
| UPH5 | [H101Y] Avastin scFv |
| UPH6 | [S105T] Avastin scFv |

**1.2 Detection of wild-type Avastin by Solution Equilibrium Titration (SET) ELISA**

**Introduction:**

Solution Equilibrium Titration (SET) ELISA was carried out on crude IVT product produced according to the method set out above for data for wild-type Avastin scFv (UPH1).

**Materials and Methods:**

In each case, wt Av scFv at a final dilution of 1:400 and Avastin at 15.625 pM final concentration were mixed with VEGF at the concentrations shown in Figure 1 (i.e., 10 nM - 0.1 pM) and allowed to reach equilibrium at room temperature overnight. Equilibrium samples were incubated for 30 min at room temperature on 96-well microtiter plates coated with 1 ug/ml VEGF (which had been blocked with 5 % (w/v) BSA 1xPBS solution for 2 hours). After washing to remove unbound material, wt Av scFv bound to the plate was probed for with an HRP labelled anti-tag antibody, Avastin was probed for with anti-Human-HRP antibody and incubated at room temperature for 1 h. ECL solution was added and signals detected immediately using a Tecan ECL platereader.

Non-linear regression for Avastin calculated using the equation for bivalent interactions:

$$Y=MAX/2/(2*A)*(-(X-(2*A)+K)+SQRT((X-(2*A)+K)^2+4*(2*A)*K)) \qquad (1)$$

Non-linear regression for UPH1 calculated using the equation for monovalent interactions:

$$Y=MAX/2/A*(-(X-A+K)+SQRT((X-A+K)^2+4*A*K)) \qquad (2)$$

where in each case Y is the measured signal level, $K=K_D$; A= starting concentration of the antibody or scFv ; X= the concentration of VEGF used in each titration step. For UPH1 A was determined by regressing against this parameter as well, using the equation above.

**Results:**

Figure 1 shows free Avastin or wt Av scFv present in equilibrium with varying concentrations of VEGF as measured by SET ELISA. Curves have been normalised to maximum (100 %) and minimum (0 %) luminescence signals. Data shown are of one experiment performed in duplicate.

$K_D$ values calculated from linear regression:

18.67 pM for Avastin when [Avastin] = 15.625 pM as determined by dilution from stock solution.

16.00 pM for UPH1.

**1.3 Expression of wild-type and mutant Avastin scFv by coupled cell-free *in vitro* transcription/translation (IVT) system**

**Introduction:**

SET ELISAs were performed on crude IVT product for each of the constructs as set out in Table 1. SET ELISA was carried out for these constructs in the same way as set out for UPH1 above.

**Materials and Methods:**

In each case, mutant Av scFv at a final dilution of 1:400 were mixed with VEGF at the concentrations shown in Figure 2 (i.e., 10 nM - 0.1 pM) and allowed to reach equilibrium at room temperature overnight. Equilibrium samples were incubated for 30 min at room temperature on 96-well microtiter plates coated with 1 ug/ml VEGF (which had been blocked with 5 % (w/v) BSA 1xPBS solution for 2 hours). After washing to remove unbound material, mutant Av scFv bound to the plate was probed for with anti-pk-HRP antibody, and incubated at room temperature for 1 h. Chemiluminescence solution was added and signals detected immediately using a chemiluminescence microplate reader.

Non-linear regression for UPH1-6 calculated using the equation (2) above for monovalent interactions.

**Results:**

Table 2. $K_D$ values calculated for UPH1-6 constructs.

| Name | Calculated $K_D$ (pM) |
|------|----------------------|
| UPH1 | 15.13 |
| UPH2 | 0.39 |
| UPH3 | 13.76 |
| UPH4 | 4.03 |
| UPH5 | 11.07 |
| UPH6 | 8.99 |

**Conclusion:**

In this small scale experiment UPH1 represents the wild-type Av scFv construct with a measured affinity approximately 15 pM, the affinity of UPH3 and UPH5 are measured to be essentially identical to that of UPH1, whilst, UPH2, 4, and 6 exhibit an increased affinity constant. All five mutations therefore appear to be permissible for introducing mutations into the construct with no loss of affinity. If an increased affinity for a mutant construct is desired then UPH2, 4, and 6 are relevant design choices.

**2. Reference Example: Investigating Kinase Activity by High-throughput Solution Titration Kinetics**

**Introduction:**

The aim of this example is the determination of the kinase activity by a high-throughput solution titration assay undertaken to identify point mutations that could be introduced in the kinase without altering or with the possibility of improving its ability to catalyze phosphorylation of a protein substrate.

**Materials and Methods:**

DNA encoding wild-type or mutant kinases are expressed using a cell-free *in vitro* coupled transcription/translation (IVT) system. A combination of site-directed mutagenesis and IVT are used to generate e.g. 100-2000 variants of the kinase (each differing from the wild-type by a single amino acid substitution). A tag in accordance with the present invention may optionally be added to the enzyme to enable later detection by ELISA.

**Results:**

Subsequent to IVT of the kinase variants, the kinase variants are combined with the kinase substrate and essential co-factors/co-substrates (i.e. ATP) in a reaction plate of a solution titration assay. A solution titration of the substrate enables the reaction rate to be monitored at a range of substrate concentrations. Reactions are initiated by the addition of the kinase. Kinases catalyze the transfer of a phosphate group from a high-energy molecule (typically ATP) to a protein substrate (Figure 3). This process is known as phosphorylation.

The amount of phosphorylated product formed is determined by a discontinuous assay. In this regard, aliquots of the enzyme reaction are taken at different predetermined time points and the formation of the phosphorylated product is determined by sandwich ELISA with antibodies directed against the reaction product e.g. the protein itself as well as the phosphorylated amino acid (Figure 4).

For the determination of the reaction velocity of the kinase, the product formation over a range of substrate concentrations at different time points is plotted over the time (Figure 5). For many enzymes, the rate of catalysis (reaction velocity, $V_0$) rises linearly as substrate concentration increases and then begins to level off and approach a maximum at higher substrate concentrations (Figure 5). Subsequently, the initial reaction velocity $V_0$ is determined from the slope of the linear part of the progress curve.

The initial velocity determined for each reaction is then plotted against the concentration of substrate titrated into the reaction [S] (Figure 6).

A hyperbolic curve results, where $V_{max}$ represents the maximum reaction velocity achieved by the system at saturating substrate concentrations [S], and the Michaelis-Menten constant, $K_M$, is the substrate concentration at which the reaction rate is half of $V_{max}$ (Figure 7). The $K_M$ gives an inverse measure of the affinity of the enzyme-substrate interaction; a lower $K_M$ value indicates a higher affinity, as the reaction will approach $V_{max}$ more rapidly. The final curve characteristics and parameters can be determined by non-linear regression of the result values obtained. In a very simple assay setup it may be the case that the reaction cannot be carried to a point where saturation begins to occur. Therefore in those situations it may be possible to determine changes in $V_0$, but not $K_M$. Nevertheless even in such a case given that the $K_M$ of the wild type enzyme is usually known, the assay can clearly show whether any particular mutant enzyme is likely to have increased, decreased or unchanged activity.

**Conclusion:**

When considering the effect(s) of point mutations in an enzyme of interest, a mutation affecting $V_{max}$ may not necessarily affect $K_M$. This is because the amino acids involved in binding the substrate may not all be involved in the catalytic reaction. Thus, a mutation may cause a drastic change in $K_M$ with little or no change in $V_{max}$ (Figure 8 A). The reverse is also possible; i.e. a mutation at/near the active site of the enzyme may affect the $V_{max}$ but not the $K_M$ if the mutated residue is not involved in substrate binding (Figure 8 B).

The ratio of these values ($V_{max}/K_m$) gives a measure of how efficiently an enzyme converts a substrate into product.

**Claims**

1. A method for screening the activity of a polypeptide comprising (i) expressing a polypeptide by coupled cell-free *in vitro* transcription-translation (IVT) and (ii) determining the activity of the expressed polypeptide by solution equilibrium titration with an interaction member, wherein the activity is the binding affinity of the polypeptide to the interaction member.

2. The method of claim 1, which is for producing a polypeptide with a desired activity.

3. The method of claim 1 or 2, further comprising introducing at least one point mutation in the amino acid sequence of the polypeptide to be expressed in step (i).

4. The method of claim 3, wherein said at least one point mutation is generated by site-directed mutagenesis.

5. The method of claim 3 or 4, wherein said at least one point mutation comprises one or more amino acid substitutions, deletions, additions, and/or insertions.

6. The method of any one of claims 1 to 5, wherein the method comprises the expression and screening of at least 100 polypeptide variants.

7. The method of any one of claims 1 to 6, wherein the polypeptide is selected from the group consisting of antibodies and fragments thereof, receptors, preferably T cell receptors, enzymes, substrates of enzymes, regulatory proteins of enzymes, cytokines, Fc fusion proteins, anticoagulants, blood factors, bone morphogenetic proteins, engineered protein scaffolds, growth factors, hormones, interferons, interleukins, and thrombolytics.

8. The method of any one of claims 1 to 7, wherein the expressed polypeptide or its interaction member contains a binding domain of an antibody fragment.

9. The method of any one of claims 1 to 7, wherein the expressed polypeptide or its interaction member contains the active domain of an enzyme, or the active domain of a substrate of an enzyme, or the active domain of a regulatory protein of an enzyme.

10. The method of any one of claims 1 to 9, wherein the screening comprises identifying a polypeptide with an affinity to an interaction memberthat is altered as a consequence of one or more introduced point mutations in said polypeptide.

11. The method of any one of claims 1 to 9, wherein the screening comprises identifying a polypeptide with an affinity to an interaction member that is not substantially altered as a consequence of one or more introduced point mutations in said polypeptide.

12. The method of any one of claims 1 to 9, wherein the screening comprises identifying a polypeptide with an affinity to an interaction member that is either increased or not substantially altered as a consequence of one or more introduced point mutations and wherein one or more of said point mutations reduces the binding of a sub-sequence of the polypeptide comprising at least one of said mutations to an MHC molecule.

13. The method of any one of claims 1 to 12, wherein screening comprises identifying a polypeptide with a cross-reactivity between more than one interaction member, and wherein the extent of such cross-reactivity is altered as a consequence of one or more introduced point mutations in said polypeptide.

14. The method of any one of claims 1 to 6, wherein the polypeptide to be screened is an enzyme, or a substrate of an enzyme or a regulatory protein of an enzyme, and wherein the activity of the polypeptide is the activity of the polypeptide in a corresponding enzyme reaction.

**Patentansprüche**

1. Verfahren zum Screenen der Aktivität eines Polypeptids, umfassend (i) Exprimieren eines Polypeptids durch gekoppelte zellfreie in *vitro*-Transkription-Translation (IVT) und (ii) Bestimmen der Aktivität des exprimierten Polypeptids durch Lösungsgleichgewicht-Titration mit einem Interaktionsteil, wobei die Aktivität die Bindungsaffinität des Polypeptids an den Interaktionsteil ist.

2. Verfahren nach Anspruch 1, das für die Herstellung eines Polypeptids mit einer gewünschten Aktivität ist.

3. Verfahren nach Anspruch 1 oder 2, des Weiteren umfassend das Einführen mindestens einer Punktmutation in die Aminosäuresequenz des in Schritt (i) zu exprimierenden Polypeptids.

4. Verfahren nach Anspruch 3, wobei die mindestens eine Punktmutation durch Site-directed Mutagenese erzeugt ist.

5. Verfahren nach Anspruch 3 oder 4, wobei die mindestens eine Punktmutation eine oder mehrere Aminosäure-Substitutionen, -Deletionen, -Hinzufügungen und/oder-Insertionen umfasst.

6. Verfahren nach einem der Ansprüche 1 bis 5, wobei das Verfahren das Exprimieren und das Screenen von mindestens 100 Polypeptid-Varianten umfasst.

7. Verfahren nach einem der Ansprüche 1 bis 6, wobei das Polypeptid ausgewählt ist aus der Gruppe bestehend aus Antikörpern und Fragmenten davon, Rezeptoren, bevorzugt T-Zell-Rezeptoren, Enzymen, Substraten von Enzymen, regulatorischen Proteinen von Enzymen, Cytokinen, Fc-Fusionsproteinen, Antikoagulantien, Blutfaktoren, knochenmorphogenetischen Proteinen (Bone Morphogenetic Proteins), gentechnisch erzeugten Proteingerüsten, Wachstumsfaktoren, Hormonen, Interferonen, Interleukinen und Thrombolytika.

8. Verfahren nach einem der Ansprüche 1 bis 7, wobei das exprimierte Polypeptid oder sein Interaktionsteil eine Bindungsdomäne eines Antikörperfragments enthält.

9. Verfahren nach einem der Ansprüche 1 bis 7, wobei das exprimierte Polypeptid oder sein Interaktionsteil die aktive Domäne eines Enzyms oder die aktive Domäne eines Substrats eines Enzyms oder die aktive Domäne eines regulatorischen Proteins eines Enzyms enthält.

10. Verfahren nach einem der Ansprüche 1 bis 9, wobei das Screenen das Identifizieren eines Polypeptids mit einer Affinität zu einem Interaktionsteil umfasst, die als Folge einer oder mehrerer eingeführter Punktmutationen in das Polypeptid verändert ist.

**11.** Verfahren nach einem der Ansprüche 1 bis 9, wobei das Screenen das Identifizieren eines Polypeptids mit einer Affinität zu einem Interaktionsteil umfasst, die als Folge einer oder mehrerer eingeführter Punktmutationen in das Polypeptid im Wesentlichen nicht verändert ist.

**12.** Verfahren nach einem der Ansprüche 1 bis 9, wobei das Screenen das Identifizieren eines Polypeptids mit einer Affinität zu einem Interaktionsteil umfasst, die als Folge einer oder mehrerer eingeführter Punktmutationen entweder erhöht oder im Wesentlichen nicht verändert ist, und wobei eine oder mehrere der Punktmutationen die Bindung einer Sub-Sequenz des Polypeptids, das mindestens eine der Mutationen umfasst, an ein MHC-Molekül verringert.

**13.** Verfahren nach einem der Ansprüche 1 bis 12, wobei das Screenen das Identifizieren eines Polypeptids mit einer Kreuzreaktivität zwischen mehr als einem Interaktionsteil umfasst, und wobei das Ausmaß einer solchen Kreuzreaktivität als Folge einer oder mehrerer eingeführter Punktmutationen in das Polypeptid verändert ist.

**14.** Verfahren nach einem der Ansprüche 1 bis 6, wobei das zu screenende Polypeptid ein Enzym oder ein Substrat eines Enzyms oder ein regulatorisches Protein eines Enzyms ist, und wobei die Aktivität des Polypeptids die Aktivität des Polypeptids in einer entsprechenden Enzymreaktion ist.

**Revendications**

**1.** Méthode pour cribler l'activité d'un polypeptide, comprenant (i) l'expression d'un polypeptide par transcription-traduction *in vitro* (IVT) acellulaire couplée et (ii) la détermination de l'activité du polypeptide exprimé par titrage à l'équilibre en solution avec un élément d'interaction, dans laquelle l'activité est l'activité de liaison du polypeptide à l'élément d'interaction.

**2.** Méthode selon la revendication 1, qui est pour produire un polypeptide ayant une activité souhaitée.

**3.** Méthode selon la revendication 1 ou 2, comprenant en outre l'introduction d'au moins une mutation ponctuelle dans la séquence d'acides aminés du polypeptide devant être exprimé dans l'étape (i).

**4.** Méthode selon la revendication 3, dans laquelle ladite au moins une mutation ponctuelle est générée par mutagenèse dirigée.

**5.** Méthode selon la revendication 3 ou 4, dans laquelle ladite au moins une mutation ponctuelle comprend une ou plusieurs substitutions, délétions, additions et/ou insertions d'acides aminés.

**6.** Méthode selon l'une quelconque des revendications 1 à 5, laquelle méthode comprend l'expression et le criblage d'au moins 100 variants polypeptidiques.

**7.** Méthode selon l'une quelconque des revendications 1 à 6, dans laquelle le polypeptide est choisi dans le groupe constitué par les anticorps et leurs fragments, les récepteurs, de préférence les récepteurs de cellules T, les enzymes, les substrats d'enzymes, les protéines régulatrices d'enzymes, les cytokines, les protéines de fusion de Fc, les anticoagulants, les facteurs sanguins, les protéines morphogénétiques osseuses, les charpentes protéiques issues du génie, les facteurs de croissance, les hormones, les interférons, les interleukines, et les thrombolytiques.

**8.** Méthode selon l'une quelconque des revendications 1 à 7, dans laquelle le polypeptide exprimé ou son élément d'interaction contient un domaine de liaison d'un fragment d'anticorps.

**9.** Méthode selon l'une quelconque des revendications 1 à 7, dans laquelle le polypeptide exprimé ou son élément d'interaction contient le domaine actif d'une enzyme, ou le domaine actif d'un substrat d'une enzyme, ou le domaine actif d'une protéine régulatrice d'une enzyme.

**10.** Méthode selon l'une quelconque des revendications 1 à 9, dans laquelle le criblage comprend l'identification d'un polypeptide ayant une affinité pour un élément d'interaction qui est altérée en conséquence d'une ou plusieurs mutations ponctuelles introduites dans ledit polypeptide.

**11.** Méthode selon l'une quelconque des revendications 1 à 9, dans laquelle le criblage comprend l'identification d'un polypeptide ayant une affinité pour un élément d'interaction qui n'est pas sensiblement altérée en conséquence d'une ou plusieurs mutations ponctuelles introduites dans ledit polypeptide.

12. Méthode selon l'une quelconque des revendications 1 à 9, dans laquelle le criblage comprend l'identification d'un polypeptide ayant une affinité pour un élément d'interaction qui est soit augmentée soit non sensiblement altérée en conséquence d'une ou plusieurs mutations ponctuelles introduites, et dans laquelle une ou plusieurs parmi lesdites mutations ponctuelles réduisent la liaison d'une sous-séquence du polypeptide comprenant au moins une desdites mutations à une molécule du MHC.

13. Méthode selon l'une quelconque des revendications 1 à 12, dans laquelle le criblage comprend l'identification d'un polypeptide ayant une réactivité croisée entre plus d'un élément d'interaction, et dans laquelle l'ampleur de cette réactivité croisée est altérée en conséquence d'une ou plusieurs mutations ponctuelles introduites dans ledit polypeptide.

14. Méthode selon l'une quelconque des revendications 1 à 6, dans laquelle le polypeptide devant être criblé est une enzyme, ou un substrat d'une enzyme ou une protéine régulatrice d'une enzyme, et dans laquelle l'activité du polypeptide est l'activité du polypeptide dans une réaction enzymatique correspondante.

[Figure 1]

[Figure 2]

[Figure 3]

[Figure 4]

[Figure 5]

[Figure 6]

$$V_o = \frac{V_{max}[S]}{K_M + [S]}$$

[Figure 7]

[Figure 8A]

[Figure 8B]

**EP 3 720 876 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 510240 A
- WO 201497113 A2
- WO 201705909 A
- EP 125023 A
- WO 9602576 A
- EP 239400 A
- US 5665563 A
- US 5492817 A
- US 5324637 A, Beckler
- US 6518058 B, Biryukov
- US 6783957 B, Biryukov
- US 20020168706 A, Chatteijee
- US 6548276 B, Swartz

- US 20040110135 A, Nemetz
- US 20040209321 A, Swartz
- US 20040214292 A, Motoda
- US 20050032086 A, Sakanyan
- US 5891637 A, Ruppert
- US 307473 A
- US 291892 A
- US 305657 A
- EP 0036776 A
- EP 0048970 A
- EP 0051873 A
- US 20050136449 A
- US 5569588 A, Ashby

**Non-patent literature cited in the description**

- **TOBIN et al.** *Curr. Drug Metab.*, 2014, vol. 15 (7), 73-756
- **POPPLEWELL**. *ADME and Translational Pharmacokinetics/Pharmacodynamics of Therapeutic Proteins*, 2015
- **NIERI et al.** *Current Clinical Medicine*, 2009, vol. 16, 753-779
- **RAJPAL et al.** *Proc. Natl. Acad. Sci.*, 2005, vol. 102, 8466-8471
- **DUBEL et al.** *Trends Biotechnology*, 2010, vol. 28, 333-339
- **IGAWA et al.** *mAbs*, 2011, vol. 3 (3), 243-252
- **BOSTROM et al.** *Science*, 2009, vol. 323, 1610-1614
- **MASON et al.** *Trends Mol. Med.*, 2002, vol. 8, 324-329
- **WURM AN BERNARD**. *Curr. Opin.Biotechnol.*, 1999, vol. 10, 156-159
- **ZOLLER**. *Cur. Opin. Biotechnol.*, vol. 2, 526-531
- **BRANNIGAN ; WILKINSON**. *Nature Rev. Mol. CEIIBiol.*, 2002, vol. 3, 964-970
- **WATSON et al.** Recombinant DNA. Scientific American Books, 1992, 453-470
- **BERGMEYER**. Grundlagen der enzymatischen Analyse. Verlag Chemie, 1977
- **BERGMEYER**. Methods of Enzymatic Analysis. Verlag Chemie, 1983
- **BISSWANGER**. Enzyme Kinetics, Principles and Methods. Wiley-VCH, 2008

- **BISSWANGER**. Practical Enzymology. Wiley-Blackwell, 2011
- **SCHOMBURG**. Springer Handbook of Enzymes. Springer, 2009
- *Brenda database*, www.brenda-enzymes.org
- *ExPASy database*, www.wxpasy.org/enzymes
- **BISSWANGER**. *Perspectives in Science*, 2014, vol. 1, 41-55
- **ACKER ; AULD**. *Perspectives in Science*, 2014, vol. 1, 56-73
- **INGLESE et al.** *Nat. Chem. Biol.*, 2007, vol. 3, 466
- **INGLESE et al.** *Nat. Chem. Biol.*, 2007, vol. 3, 438
- **FANG et al.** *Trends in Bio/Pharmaceutical Industry.*, 2007, vol. 3, 34-8
- **RICH ; MYSKA**. *Analytical Biochemistry.*, 2005, vol. 361 (1), 1-6
- **HEMERHORST et al.** *Clin Biochem Rev*, 2012, vol. 33 (4), 161-173
- **DAY et al.** *Protein Sci*, 2002, vol. 11, 1017-1025
- **MYSZKA et al.** *J Biomol Tech*, 2003, vol. 14, 247-269
- **BEE et al.** *PLOS ONE*, 2012, vol. 7, e36261
- **DRAKE et al.** *Anal Biochem*, 2004, vol. 328, 35-43
- **ABDICHE et al.** *Anal Biochem*, 2011, vol. 411, 139-151
- **DRAKE et al.** *Anal Biochem*, 2012, vol. 429, 58-69

34

- **WURM** ; **BERNARD**. *Curr. Opin.Biotechnol.*, 1999, vol. 10, 156-159
- **BRANNIGAN** ; **WILKINSON**. *Nature Rev. Mol. Cell Biol.*, 2002, vol. 3, 964-970
- **CARLSON et al.** *Biotechnol. Adv.*, 2012, vol. 30 (5), 1185-94
- **OHLMANN et al.** *EMBO J.*, 1997, vol. 16 (4), 844-855
- **OHLMANN et al.** *J Mol Biol.*, 2002, vol. 318 (1), 9-20
- **ZIEGLER et al.** *Virology*, 1995, vol. 213 (2), 549-557
- **ZIEGLER et al.** *J Virol.*, 1995, vol. 69 (6), 3465-3474
- **GOSHIMA et al.** *Nat Methods*, 2008, vol. 5 (12), 1011-1017
- **LU et al.** *Synthetic and Systems Biotechnology*, 2017, vol. 2 (1), 23-27
- **DELLA DUCATA et al.** *Journal of Biomolecular Screening*, 2015, vol. 20 (10), 1256-1267
- **FRIGUET B et al.** Protein Structure, A Practical Approach. 1997, 335
- **HUBER**. *J. Struct. Biol.*, 2007, vol. 159, 206-221
- **YIN**. *mAbs*, 2012, vol. 4 (2), 217-225
- **ANGAG** ; **SCHUTZ**. *Biotech*, 2001, vol. 30, 486-488
- **WANG** ; **WILKINSON**. *Biotech*, 2000, vol. 29, 976-978
- **KANG et al.** *Biotech.*, 1996, vol. 20, 44-46
- **OGEL** ; **MCPHERSON**. *Protein Engineer.*, 1992, vol. 5, 467-468
- **KIRSCH** ; **JOLY**. *Nuc. Acids. Res.*, 1998, vol. 26, 1848-1850
- **RHEM** ; **HANCOCK**. *J. Bacteriol.*, 1996, vol. 178, 3346-3349
- **BOLES** ; **MIOGSA**. *Curr. Genet.*, 1995, vol. 28, 197-198
- **BARRENTTINO et al.** *Nuc. Acids. Res.*, 1993, vol. 22, 541-542
- **TESSIER** ; **THOMAS**. *Meths. Molec. Biol.*, vol. 57, 229-237
- **PONS et al.** *Meth. Molec. Biol.*, vol. 67, 209-218
- **SANGER** ; **COULSON**. *J. Mol. Biol.*, 1975, vol. 94 (3), 41-448
- **SANGER et al.** *Proc. Natl Acad. Sci*, 1977, vol. 74 (12), 5463-5467
- **MARDIS**. *Annu. Rev. Genomics Hum. Genet.*, 2008, vol. 9, 387-402
- ; **METZKER**. *Nat. Rev. Genet.*, 2010, vol. 11, 31-46
- **SHENDURE** ; **JI**. *Nat. Biotechnol.*, 2008, vol. 26, 1135-1145
- **KULSKI et al.** *Intech*, 2014, 141-481
- **KOZAREVA et al.** *Nat. Methods*, 2009, vol. 6, 291-295
- **LIE et al.** *Curr Opin Biotechnol*, 1998, vol. 9, 43-8
- **TAN et al.** *Chemistry*, 2000, vol. 6, 1107-11
- **LAAN et al.** *Hum Genet*, 1995, vol. 96, 275-80
- **FOROZAN et al.** *Trends Genet*, 1997, vol. 13, 405-9
- **LIZARDI et al.** *Nat Genet*, 1998, vol. 19, 225-32
- **SOUTHERN**. *J. Mol. Biol.*, 1998, vol. 98, 503
- **MORRISON et al.** *Proc. Nat'l Acad. Sci.*, 1984, vol. 81, 6851
- **HOOGENBOOM et al.** *Immunol Today*, 2000, vol. 21 (8), 371-8
- **GAVILONDO et al.** *Biotechniques*, 2000, vol. 29 (1), 128-32
- **IBA**. *Immunol. Cell Biol.*, 1997, vol. 75, 217-221
- **MARKS, N.** *Engl. J. Med*, 1996, vol. 335, 730-733
- **CO, M.S. et al.** *J. Immunol.*, 1994, vol. 152, 2968-2976
- **BETTER, M.** ; **HORWITZ, A.H.** *Methods in Enzymology*, 1989, vol. 178, 476-496
- **PLUECKTHUN, A.** ; **SKERRA, A.** *Methods in Enzymology*, 1989, vol. 178, 476-496
- **LAMOYI, E.** *Methods in Enzymology*, 1989, vol. 121, 652-663
- **ROUSSEAUX, J. et al.** *Methods in Enzymology*, 1989, vol. 121, 663-669
- **BIRD, R.E. et al.** *TIBTECH*, 1991, vol. 9, 132-137
- **HUSTON, J.S. et al.** *Proc. Natl. Acad. Sci. U. S. A.*, 1988, vol. 85, 5879-5883
- **PLUECKTHUN**. The Pharmacology of Monoclonal Antibodies. Springer Verlag, 1994, vol. 113, 269-315
- **SKERRA**. Engineered protein scaffolds for molecular recognition. *J. Mol. Recognit*, 2000, vol. 13, 167-187
- Construction of Recombinant DNA Libraries. Current Protocols in Molecular Biology. John Wiley & Sons, 1988
- Preparation of RNA from Eukaryotic and Prokaryotic Cells.. Current Protocols in Molecular Biology. John Wiley & Sons, 1988
- *Nucleic Acids Res.*, 1990, vol. 18 (17), 5322
- **HOLMES** ; **QUIGLEY**. *Anal. Biochem.*, 1981, vol. 114, 193
- **BIRNBOIM** ; **DOLY**. *Nuc. Acids Res.*, 1979, vol. 7, 1513
- **GODSON** ; **VAPNEK**. *Biochem. Biophys. Acta*, 1973, vol. 299, 516
- **BIRNBOIM, H. C** ; **DOLY, J.** *Nucl. Acids Res.*, 1979, vol. 7, 1513
- **PELHAM et al.** *European Journal of Biochemistry*, 1976, vol. 67, 247
- **KIM et al.** *Biotechnol Bioeng.*, 2001, vol. 74 (4), 309-16

- **BURNETTE**. *A. Anal. Biochem.*, 1981, vol. 112, 195-203
- **THORSNESS et al.** *Mol. Cell. Biol*, 1989, vol. 9, 5702-5712
- **CYERT et al.** *Mol. Cell. Biol.*, 1992, vol. 12, 3460-3469
- **SCHENA et al.** Quantitative monitoring of gene expression patterns with a complementary DNA micro-array. *Science*, 1995, vol. 270, 467-470
- **LOCKHORT et al.** Expression monitoring by hybridization to high-density oligonucleotide arrays. *Nature Biotechnology*, 1996, vol. 14, 1675-1680
- **BLANCHARD et al.** Sequence to array: Probing the genome's secrets. *Nature Biotechnology*, 1996, vol. 14, 1649
- Strategies for Protein Purification and Characterization: A Laboratory Course Manual.. Cold Spring Harbor Laboratory Press, 1996
- **GUSTCHINA et al.** *Virology*, 2009, vol. 393, 112-119
- **HUBER et al.** *J. Struct. Biol.*, 2007, vol. 159, 206-221
- **HAENEL**. *Anal. Biochem.*, 2005, vol. 339, 182-184
- **LAD et al.** *J. Biomol. Screen.*, 2015, vol. 20, 498-507
- **YLERA et al.** *Anal. Biochem.*, 2013, vol. 441, 208-213
- **CANZIANI et al.** *Anal. Biochem.*, 2004, vol. 325, 301-307
- *Wikipedia article*, https://en.wikipedia.org/wiki/Dissociation constant
- **GOTCH et al.** *J Exp Med.*, 1988, vol. 168 (6), 2045-57
- **PETROVA et al.** *J Immunol.*, 2011, vol. 186 (11), 6390-7
- **KAN-MITCHELL et al.** *J Immunol.*, 2006, vol. 176 (11), 6690-701
- **VALI et al.** *J Virol.*, 2011, vol. 85 (1), 254-63
- **ACIERNO et al.** *J Transl Med.*, 2003, vol. 1 (1), 3
- **WEDEMEYER et al.** *J Virol.*, 2001, vol. 75 (23), 11392-400
- **KASPROWICZ et al.** *J Clin Invest.*, 2008, vol. 118 (3), 1143-53
- **HINTON et al.** *J Immunol.*, 2005, vol. 176 (1), 346-56
- **DALL'ACQUA et al.** *J. Biol. Chem.*, 2006, vol. 281 (33), 23514-24
- **JOHANSSON et al.** *J. Biol. Chem*, 2002, vol. 277, 8114-8120
- **LINHULT et al.** *Protein Sci.*, 2002, vol. 11, 206-213
- **ZALEVSKY et al.** *Nat. Biotechnol.*, 2010, vol. 28 (2), 157-159
- **KIM et al.** *Eur J Immunol.*, 1994, vol. 24, 2429
- **KUO et al.** *Journal of Clinical Immunology.*, 2010, vol. 30 (6), 777-789
- **ZALEVSKY et al.** *nature Biotechnology*, 2010, vol. 28 (2), 157-159